# EUROPEAN PATENT APPLICATION

(11) **EP 2 072 527 A1**
(43) Date of publication of application: **24.06.2009**
(21) Application number: 07123906.5
(22) Date of filing: 21.12.2007
(51) Int. Cl.: C07K 14/435, C07K 19/00, C12N 15/12, C12N 15/62

(54) **Fusion polypeptides comprising a SHBG dimerization component and uses thereof**

(71) Applicant: Altonabiotec AG, 202051 Hamburg (DE)
(72) Inventor: Krietsch, Thomas, Dr., 22459 Hamburg (DE); Hunt, Nicholas, Dr., 21629 Neu Wulmstorf (DE); Engeland, Birgit, Dr., 22391 Hamburg (DE); Heibey, Monika, 22523 Hamburg (DE)
(74) Representative: Meyers, Hans-Wilhelm

(57) **Abstract**

The present invention relates to fusion polypeptides comprising a biologically active component and a dimerization component, wherein said dimerization component is derived from sex hormone-binding globuline (SHBG). The invention further relates to nucleic acid molecules encoding the fusion polypeptides and to use of these polypeptides for treatment of various diseases.

SEQUENCE LISTING

<110>  Altonabiotec AG

<120>  FUSION POLYPEPTIDES COMPRISING A SHBG DIMERIZATION DOMAIN AND
       USES THEREOF

<130>  P74723

<160>  95

<170>  PatentIn version 3.4

<210>  1
<211>  615
<212>  DNA
<213>  Homo sapiens

<400>  1
gataccaacc ctaaggatga ctggtttatg ctgggacttc gagacggcag gcctgagatc      60

caactgcaca atcactgggc ccagcttacg gtgggtgctg gaccacggct ggatgatggg     120

agatggcacc aggtggaagt caagatggag ggggactctg tgctgctgga ggtggatggg     180

gaggaggtgc tgcgcctgag acaggtctct gggcccctga ccagcaaacg ccatcccatc     240

atgaggattg cgcttggggg gctgctcttc cccgcttcca accttcggtt gccgctggtt     300

cctgccctgg atggctgcct gcgccgggat tcctggctgg acaaacaggc cgagatctca     360

gcatctgccc ccactagcct cagaagctgt gatgtagaat caaatcccgg gatatttctc     420

cctccaggga ctcaggcaga attcaatctc cgagacattc cccagcctca tgcagagccc     480

tgggccttct ctttggacct gggactcaag caggcagcag gctcaggcca cctccttgct     540

cttgggacac cagagaaccc atcttggctc agtctccacc tccaagatca aaaggtggtg     600

ttgtcttctg ggtcg                                                      615

<210>  2
<211>  205
<212>  PRT
<213>  Homo sapiens

<400>  2

Asp Thr Asn Pro Lys Asp Asp Trp Phe Met Leu Gly Leu Arg Asp Gly
1               5                   10                  15

Arg Pro Glu Ile Gln Leu His Asn His Trp Ala Gln Leu Thr Val Gly
            20                  25                  30

Ala Gly Pro Arg Leu Asp Asp Gly Arg Trp His Gln Val Glu Val Lys
        35                  40                  45

Met Glu Gly Asp Ser Val Leu Leu Glu Val Asp Gly Glu Glu Val Leu
    50                  55                  60

Arg Leu Arg Gln Val Ser Gly Pro Leu Thr Ser Lys Arg His Pro Ile
65                  70                  75                  80

Met Arg Ile Ala Leu Gly Gly Leu Leu Phe Pro Ala Ser Asn Leu Arg
                85                  90                  95

Leu Pro Leu Val Pro Ala Leu Asp Gly Cys Leu Arg Arg Asp Ser Trp
            100                 105                 110

Leu Asp Lys Gln Ala Glu Ile Ser Ala Ser Ala Pro Thr Ser Leu Arg
        115                 120                 125

Ser Cys Asp Val Glu Ser Asn Pro Gly Ile Phe Leu Pro Pro Gly Thr
    130                 135                 140

Gln Ala Glu Phe Asn Leu Arg Asp Ile Pro Gln Pro His Ala Glu Pro
145                 150                 155                 160

Trp Ala Phe Ser Leu Asp Leu Gly Leu Lys Gln Ala Ala Gly Ser Gly
                165                 170                 175

His Leu Leu Ala Leu Gly Thr Pro Glu Asn Pro Ser Trp Leu Ser Leu
            180                 185                 190

His Leu Gln Asp Gln Lys Val Val Leu Ser Ser Gly Ser
        195                 200                 205

<210>  3
<211>  168
<212>  DNA
<213>  Homo sapiens

<400>  3
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgc 168

<210> 4
<211> 56
<212> PRT
<213> Homo sapiens

<400> 4

Ser Asp Thr Val Cys Asp Ser Cys Glu Asp Ser Thr Tyr Thr Gln Leu
1               5                   10                  15

Trp Asn Trp Val Pro Glu Cys Leu Ser Cys Gly Ser Arg Cys Ser Ser
            20                  25                  30

Asp Gln Val Glu Thr Gln Ala Cys Thr Arg Glu Gln Asn Arg Ile Cys
        35                  40                  45

Thr Cys Arg Pro Gly Trp Tyr Cys
    50                  55

<210> 5
<211> 171
<212> DNA
<213> Homo sapiens

<400> 5
tcggacaccg tgtgtgactc ctgtgaggac agcacataca cccagctctg gaactgggtt 60

cccgagtgct tgagctgtgg ctcccgctgt agctctgacc aggtggaaac tcaagcctgc 120

actcgggaac agaaccgcat ctgcacctgc aggcccggct ggtactgcgc g 171

<210> 6
<211> 174
<212> DNA
<213> Homo sapiens

<400> 6
tcggacaccg tgtgtgactc ctgtgaggac agcacataca cccagctctg gaactgggtt 60

cccgagtgct tgagctgtgg ctcccgctgt agctctgacc aggtggaaac tcaagcctgc 120

actcgggaac agaaccgcat ctgcacctgc aggcccggct ggtactgcgc gctg 174

<210> 7
<211> 177
<212> DNA
<213> Homo sapiens

<400>  7
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagc        177


<210>  8
<211>  180
<212>  DNA
<213>  Homo sapiens

<400>  8
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180


<210>  9
<211>  183
<212>  DNA
<213>  Homo sapiens

<400>  9
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

cag                                                                   183


<210>  10
<211>  186
<212>  DNA
<213>  Homo sapiens

<400>  10
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggag                                                                186


<210>  11
<211>  189
<212>  DNA
<213>  Homo sapiens

<400>  11
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggagggg                                                             189


<210>  12
<211>  192
<212>  DNA
<213>  Homo sapiens

<400>  12
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggaggggt gc                                                         192


<210>  13
<211>  195
<212>  DNA
<213>  Homo sapiens

<400>  13
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggaggggt gccgg                                                      195


<210>  14
<211>  198
<212>  DNA
<213>  Homo sapiens

<400>  14
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggaggggt gccggctg                                                   198

<210>  15
<211>  201
<212>  DNA
<213>  Homo sapiens

<400>  15
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggaggggt gccggctgtg c                                               201


<210>  16
<211>  204
<212>  DNA
<213>  Homo sapiens

<400>  16
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggaggggt gccggctgtg cgcg                                            204


<210>  17
<211>  207
<212>  DNA
<213>  Homo sapiens

<400>  17
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggaggggt gccggctgtg cgcgccg                                         207


<210>  18
<211>  210
<212>  DNA
<213>  Homo sapiens

<400>  18
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggaggggt gccggctgtg cgcgccgctg                                    210


<210>  19
<211>  213
<212>  DNA
<213>  Homo sapiens

<400>  19
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggaggggt gccggctgtg cgcgccgctg cgc                                  213


<210>  20
<211>  216
<212>  DNA
<213>  Homo sapiens

<400>  20
tcggacaccg tgtgtgactc ctgtgaggac agcacataca cccagctctg gaactgggtt      60

cccgagtgct tgagctgtgg ctcccgctgt agctctgacc aggtggaaac tcaagcctgc     120

actcgggaac agaaccgcat ctgcacctgc aggcccggct ggtactgcgc gctgagcaag     180

caggaggggt gccggctgtg cgcgccgctg cgcaag                               216


<210>  21
<211>  87
<212>  DNA
<213>  Artificial

<220>
<223>  Nucleic acid encoding Ig hinge linker

<400>  21
tgcgacaaga cccacacctg ccccccctgc cccgcccccg agctgctggg cggcccctcc      60

gtgttcctgt tcccccccaa gcccaag                                          87


<210>  22
<211>  29
<212>  PRT
<213>  Artificial

<220>
<223>  Ig hinge linker

<400>  22

Cys Asp Lys Thr His Thr Cys Pro Pro Cys Pro Ala Pro Glu Leu Leu
1               5                   10                  15

Gly Gly Pro Ser Val Phe Leu Phe Pro Pro Lys Pro Lys
            20                  25

<210>  23
<211>  20
<212>  DNA
<213>  Artificial

<220>
<223>  Oligonucleotide primer

<400>  23
aacccatatg gataccaacc                                                   20

<210>  24
<211>  23
<212>  DNA
<213>  Artificial

<220>
<223>  Oligonucleotide primer

<400>  24
ggttggatcc tcacgaccca gaa                                               23

<210>  25
<211>  20
<212>  DNA
<213>  Artificial

<220>
<223>  Oligonucleotide primer

<400>  25
ccaaatgggg gagtgagagg                                                   20

<210>  26
<211>  22
<212>  DNA
<213>  Artificial

<220>
<223>  Oligonucleotide primer

<400>  26
tatgtacatc gaggggttag ca                                                22

<210>  27
<211>  17
<212>  DNA
<213>  Artificial

<220>
<223>  Oligonucleotide primer

<400>  27
gagggcaggg ggcaacc                                                      17


<210>  28
<211>  18
<212>  DNA
<213>  Artificial

<220>
<223>  Oligonucleotide primer

<400>  28
ggcggggcag gtcacaga                                                     18


<210>  29
<211>  1544
<212>  DNA
<213>  Homo sapiens

<400>  29
ccaaatgggg gagtgagagg ccatagctgt ctggcatggg cctctccacc gtgcctgacc      60

tgctgctgcc actggtgctc ctggagctgt tggtgggaat atacccctca ggggttattg     120

gactggtccc tcacctaggg gacagggaga agagagatag tgtgtgtccc caaggaaaat     180

atatccaccc tcaaaataat tcgatttgct gtaccaagtg ccacaaagga acctacttgt     240

acaatgactg tccaggcccg gggcaggata cggactgcag ggagtgtgag agcggctcct     300

tcaccgcttc agaaaaccac ctcagacact gcctcagctg ctccaaatgc cgaaaggaaa     360

tgggtcaggt ggagatctct tcttgcacag tggaccggga caccgtgtgt ggctgcagga     420

agaaccagta ccggcattat tggagtgaaa accttttcca gtgcttcaat tgcagcctct     480

gcctcaatgg gaccgtgcac ctctcctgcc aggagaaaca gaacaccgtg tgcacctgcc     540

atgcaggttt ctttctaaga gaaaacgagt gtgtctcctg tagtaactgt aagaaaagcc     600

tggagtgcac gaagttgtgc ctaccccaga ttgagaatgt taagggcact gaggactcag     660

gcaccacagt gctgttgccc ctggtcattt tctttggtct ttgcctttta tccctcctct     720

tcattggttt aatgtatcgc taccaacggt ggaagtccaa gctctactcc attgtttgtg 780

ggaaatcgac acctgaaaaa gagggggagc ttgaaggaac tactactaag cccctggccc 840

caaacccaag cttcagtccc actccaggct tcacccccac cctgggcttc agtcccgtgc 900

ccagttccac cttcacctcc agctccacct atacccccgg tgactgtccc aactttgcgg 960

ctccccgcag agaggtggca ccaccctatc agggggctga ccccatcctt gcgacagccc 1020

tcgcctccga ccccatcccc aacccccttc agaagtggga ggacagcgcc cacaagccac 1080

agagcctaga cactgatgac cccgcgacgc tgtacgccgt ggtggagaac gtgcccccgt 1140

tgcgctggaa ggaattcgtg cggcgcctag ggctgagcga ccacgagatc gatcggctgg 1200

agctgcagaa cgggcgctgc ctgcgcgagg cgcaatacag catgctggcg acctggaggc 1260

ggcgcacgcc gcggcgcgag gccacgctgg agctgctggg acgcgtgctc cgcgacatgg 1320

acctgctggg ctgcctggag gacatcgagg aggcgctttg cggccccgcc gccctcccgc 1380

ccgcgcccag tcttctcaga tgaggctgcg cccctgcggg cagctctaag gaccgtcctg 1440

cgagatcgcc ttccaacccc acttttttct ggaaaggagg ggtcctgcag gggcaagcag 1500

gagctagcag ccgcctactt ggtgctaacc cctcgatgta cata 1544

<210> 30
<211> 1718
<212> DNA
<213> Homo sapiens

<400> 30
gagggcaggg ggcaaccgga ccccgcccgc acccatggcg cccgtcgccg tctgggccgc 60

gctggccgtc ggactggagc tctgggctgc ggcgcacgcc ttgcccgccc aggtggcatt 120

tacaccctac gccccggagc ccgggagcac atgccggctc agagaatact atgaccagac 180

agctcagatg tgctgcagca aatgctcgcc gggccaacat gcaaaagtct tctgtaccaa 240

gacctcggac accgtgtgtg actcctgtga ggacagcaca tacacccagc tctggaactg 300

ggttcccgag tgcttgagct gtggctcccg ctgtagctct gaccaggtgg aaactcaagc 360

ctgcactcgg gaacagaacc gcatctgcac ctgcaggccc ggctggtact gcgcgctgag 420

caagcaggag gggtgccggc tgtgcgcgcc gctgcgcaag tgccgcccgg gcttcggcgt 480

ggccagacca ggaactgaaa catcagacgt ggtgtgcaag ccctgtgccc cggggacgtt 540

ctccaacacg acttcatcca cggatatttg caggccccac cagatctgta acgtggtggc 600

catccctggg aatgcaagca tggatgcagt ctgcacgtcc acgtccccca cccggagtat 660

ggccccaggg gcagtacact taccccagcc agtgtccaca cgatcccaac acacgcagcc 720

aactccagaa cccagcactg ctccaagcac ctccttcctg ctcccaatgg gccccagccc 780

cccagctgaa gggagcactg gcgacttcgc tcttccagtt ggactgattg tgggtgtgac 840

agccttgggt ctactaataa taggagtggt gaactgtgtc atcatgaccc aggtgaaaaa 900

gaagcccttg tgcctgcaga gagaagccaa ggtgcctcac ttgcctgccg ataaggcccg 960

gggtacacag ggccccgagc agcagcacct gctgatcaca gcgccgagct ccagcagcag 1020

ctccctggag agctcggcca gtgcgttgga cagaagggcg cccactcgga accagccaca 1080

ggcaccaggc gtggaggcca gtggggccgg ggaggcccgg gccagcaccg ggagctcaga 1140

ttcttcccct ggtggccatg ggacccaggt caatgtcacc tgcatcgtga acgtctgtag 1200

cagctctgac cacagctcac agtgctcctc ccaagccagc tccacaatgg gagacacaga 1260

ttccagcccc tcggagtccc cgaaggacga gcaggtcccc ttctccaagg aggaatgtgc 1320

ctttcggtca cagctggaga cgccagagac cctgctgggg agcaccgaag agaagcccct 1380

gccccttgga gtgcctgatg ctgggatgaa gcccagttaa ccaggccggt gtgggctgtg 1440

tcgtagccaa ggtgggctga gccctggcag gatgaccctg cgaaggggcc ctggtccttc 1500

caggccccca ccactaggac tctgaggctc tttctgggcc aagttcctct agtgccctcc 1560

acagccgcag cctccctctg acctgcaggc caagagcaga ggcagcgagt tgtggaaagc 1620

ctctgctgcc atggcgtgtc cctctcggaa ggctggctgg gcatggacgt tcggggcatg 1680

ctggggcaag tccctgactc tctgtgacct gccccgcc 1718

<210> 31
<211> 324
<212> DNA
<213> Homo sapiens

<400> 31
gatagtgtgt gtccccaagg aaaatatatc caccctcaaa ataattcgat ttgctgtacc 60

aagtgccaca aaggaaccta cttgtacaat gactgtccag gcccggggca ggatacggac 120

tgcagggagt gtgagagcgg ctccttcacc gcttcagaaa accacctcag acactgcctc 180

agctgctcca aatgccgaaa ggaaatgggt caggtggaga tctcttcttg cacagtggac 240

cgggacaccg tgtgtggctg caggaagaac cagtaccggc attattggag tgaaaacctt 300

ttccagtgct tcaattgcag cctc 324

<210>  32
<211>  381
<212>  DNA
<213>  Homo sapiens

<400>  32
gatagtgtgt gtccccaagg aaaatatatc caccctcaaa ataattcgat ttgctgtacc      60

aagtgccaca aaggaaccta cttgtacaat gactgtccag gcccggggca ggatacggac     120

tgcagggagt gtgagagcgg ctccttcacc gcttcagaaa accacctcag acactgcctc     180

agctgctcca aatgccgaaa ggaaatgggt caggtggaga tctcttcttg cacagtggac     240

cgggacaccg tgtgtggctg caggaagaac cagtaccggc attattggag tgaaaacctt     300

ttccagtgct tcaattgcag cctctgcctc aatgggaccg tgcacctctc ctgccaggag     360

aaacagaaca ccgtgtgcac c                                               381


<210>  33
<211>  483
<212>  DNA
<213>  Homo sapiens

<400>  33
gatagtgtgt gtccccaagg aaaatatatc caccctcaaa ataattcgat ttgctgtacc      60

aagtgccaca aaggaaccta cttgtacaat gactgtccag gcccggggca ggatacggac     120

tgcagggagt gtgagagcgg ctccttcacc gcttcagaaa accacctcag acactgcctc     180

agctgctcca aatgccgaaa ggaaatgggt caggtggaga tctcttcttg cacagtggac     240

cgggacaccg tgtgtggctg caggaagaac cagtaccggc attattggag tgaaaacctt     300

ttccagtgct tcaattgcag cctctgcctc aatgggaccg tgcacctctc ctgccaggag     360

aaacagaaca ccgtgtgcac ctgccatgca ggtttctttc taagagaaaa cgagtgtgtc     420

tcctgtagta actgtaagaa aagcctggag tgcacgaagt tgtgcctacc ccagattgag     480

aat                                                                   483


<210>  34
<211>  558
<212>  DNA
<213>  Homo sapiens

<400>  34
atggcgcccg tcgccgtctg ggccgcgctg gccgtcggac tggagctctg ggctgcggcg      60

cacgccttgc ccgcccaggt ggcatttaca ccctacgccc cggagcccgg gagcacatgc     120

cggctcagag aatactatga ccagacagct cagatgtgct gcagcaaatg ctcgccgggc    180

caacatgcaa aagtcttctg taccaagacc tcggacaccg tgtgtgactc ctgtgaggac    240

agcacataca cccagctctg gaactgggtt cccgagtgct tgagctgtgg ctcccgctgt    300

agctctgacc aggtggaaac tcaagcctgc actcgggaac agaaccgcat ctgcacctgc    360

aggcccggct ggtactgcgc gctgagcaag caggaggggt gccggctgtg cgcgccgctg    420

cgcaagtgcc gcccgggctt cggcgtggcc agaccaggaa ctgaaacatc agacgtggtg    480

tgcaagccct gtgccccggg gacgttctcc aacacgactt catccacgga tatttgcagg    540

ccccaccaga tctgttaa                                                  558

<210>  35
<211>  26
<212>  DNA
<213>  Artificial

<220>
<223>  Oligonucleotide primer

<400>  35
aacccatatg gatagtgtgt gtcccc                                          26

<210>  36
<211>  30
<212>  DNA
<213>  Artificial

<220>
<223>  Oligonucleotide primer

<400>  36
ggttggatcc tcagaggctg caattgaagc                                      30

<210>  37
<211>  29
<212>  DNA
<213>  Artificial

<220>
<223>  Oligonucleotide primer

<400>  37
ggttggatcc tcaggtgcac acggtgttc                                       29

<210>  38
<211>  31

<212>  DNA
<213>  Artificial

<220>
<223>  Oligonucleotide primer

<400>  38
ggttggatcc tcaattctca atctggggta g                                    31


<210>  39
<211>  30
<212>  DNA
<213>  Artificial

<220>
<223>  Oligonucleotide primer

<400>  39
aaccaagctt ccaccatggg cctctccacc                                      30


<210>  40
<211>  30
<212>  DNA
<213>  Artificial

<220>
<223>  Oligonucleotide primer

<400>  40
ggttggatcc tcagaggctg caattgaagc                                      30


<210>  41
<211>  29
<212>  DNA
<213>  Artificial

<220>
<223>  Oligonucleotide primer

<400>  41
ggttggatcc tcaggtgcac acggtgttc                                       29


<210>  42
<211>  31
<212>  DNA
<213>  Artificial

<220>
<223>  Oligonucleotide primer

<400>  42
ggttggatcc tcaattctca atctggggta g                                    31

<210>  43
<211>  31
<212>  DNA
<213>  Artificial

<220>
<223>  Oligonucleotide primer

<400>  43
aactcgaggc cgccaccatg gcgcccgtcg c                                    31


<210>  44
<211>  26
<212>  DNA
<213>  Artificial

<220>
<223>  Oligonucleotide primer

<400>  44
ttgcggccgc ttaacagatc tggtgg                                          26


<210>  45
<211>  26
<212>  DNA
<213>  Artificial

<220>
<223>  Oligonucleotide primer

<400>  45
aacccatatg tcggacaccg tgtgtg                                          26


<210>  46
<211>  30
<212>  DNA
<213>  Artificial

<220>
<223>  Oligonucleotide primer

<400>  46
ggttggatcc tcagcagtac cagccgggcc                                      30


<210>  47
<211>  99
<212>  DNA
<213>  Artificial

<220>

<223>  Oligonucleotide primer

<400>  47
aactcgaggc cgccaccatg gcgcccgtcg ccgtctgggc cgcgctggcc gtcggactgg      60

agctctgggc tgcggcgcac gcctcggaca ccgtgtgtg                             99


<210>  48
<211>  30
<212>  DNA
<213>  Artificial

<220>
<223>  Oligonucleotide primer

<400>  48
ttgcggccgc tcagcagtac cagccgggcc                                       30


<210>  49
<211>  30
<212>  DNA
<213>  Artificial

<220>
<223>  Oligonucleotide primer

<400>  49
gggtcttgtc gcagaggctg caattgaagc                                       30


<210>  50
<211>  26
<212>  DNA
<213>  Artificial

<220>
<223>  Oligonucleotide primer

<400>  50
gggtcttgtc gcaggtgcac acggtg                                           26


<210>  51
<211>  28
<212>  DNA
<213>  Artificial

<220>
<223>  Oligonucleotide primer

<400>  51
gggtcttgtc gcaattctca atctgggg                                         28

<210>  52
<211>  27
<212>  DNA
<213>  Artificial

<220>
<223>  Oligonucleotide sequence

<400>  52
cttagggttg gtatccttgg gcttggg                                          27


<210>  53
<211>  27
<212>  DNA
<213>  Artificial

<220>
<223>  Oligonucleotide primer

<400>  53
cccaagccca aggataccaa ccctaag                                          27


<210>  54
<211>  31
<212>  DNA
<213>  Artificial

<220>
<223>  Oligonucleotide primer

<400>  54
ggttggatcc tcacgaccca gaagacaaca c                                     31


<210>  55
<211>  1032
<212>  DNA
<213>  Artificial

<220>
<223>  Pro_TNFaR1a D41-L108_hinge_SHBG construct

<400>  55
atggatagtg tgtgtcccca aggaaaatat atccaccctc aaaataattc gatttgctgt      60

accaagtgcc acaaaggaac ctacttgtac aatgactgtc caggcccggg gcaggatacg     120

gactgcaggg agtgtgagag cggctccttc accgcttcag aaaaccacct cagacactgc     180

ctcagctgct ccaaatgccg aaaggaaatg ggtcaggtgg agatctcttc ttgcacagtg     240

gaccgggaca ccgtgtgtgg ctgcaggaag aaccagtacc ggcattattg gagtgaaaac     300

cttttccagt gcttcaattg cagcctctgc gacaagaccc acacctgccc cccctgcccc     360

gcccccgagc tgctgggcgg cccctccgtg ttcctgttcc cccccaagcc caaggatacc     420

aaccctaagg atgactggtt tatgctggga cttcgagacg gcaggcctga gatccaactg     480

cacaatcact gggcccagct tacggtgggt gctggaccac ggctggatga tgggagatgg     540

caccaggtgg aagtcaagat ggagggggac tctgtgctgc tggaggtgga tggggaggag     600

gtgctgcgcc tgagacaggt ctctgggccc ctgaccagca aacgccatcc catcatgagg     660

attgcgcttg gggggctgct cttccccgct tccaaccttc ggttgccgct ggttcctgcc     720

ctggatggct gcctgcgccg ggattcctgg ctggacaaac aggccgagat ctcagcatct     780

gcccccacta gcctcagaag ctgtgatgta gaatcaaatc ccgggatatt tctccctcca     840

gggactcagg cagaattcaa tctccgagac attccccagc ctcatgcaga gccctgggcc     900

ttctctttgg acctgggact caagcaggca gcaggctcag gccacctcct tgctcttggg     960

acaccagaga acccatcttg gctcagtctc cacctccaag atcaaaaggt ggtgttgtct    1020

tctgggtcgt ga                                                        1032

<210>  56
<211>  343
<212>  PRT
<213>  Artificial

<220>
<223>  Pro_TNFaR1a D41-L108_hinge_SHBG protein

<400>  56

Met Asp Ser Val Cys Pro Gln Gly Lys Tyr Ile His Pro Gln Asn Asn
1               5                   10                  15

Ser Ile Cys Cys Thr Lys Cys His Lys Gly Thr Tyr Leu Tyr Asn Asp
            20                  25                  30

Cys Pro Gly Pro Gly Gln Asp Thr Asp Cys Arg Glu Cys Glu Ser Gly
        35                  40                  45

Ser Phe Thr Ala Ser Glu Asn His Leu Arg His Cys Leu Ser Cys Ser
    50                  55                  60

Lys Cys Arg Lys Glu Met Gly Gln Val Glu Ile Ser Ser Cys Thr Val
65                  70                  75                  80

Asp Arg Asp Thr Val Cys Gly Cys Arg Lys Asn Gln Tyr Arg His Tyr
                85                  90                  95

Trp Ser Glu Asn Leu Phe Gln Cys Phe Asn Cys Ser Leu Cys Asp Lys
            100                 105                 110

Thr His Thr Cys Pro Pro Cys Pro Ala Pro Glu Leu Leu Gly Gly Pro
        115                 120                 125

Ser Val Phe Leu Phe Pro Pro Lys Pro Lys Asp Thr Asn Pro Lys Asp
    130                 135                 140

Asp Trp Phe Met Leu Gly Leu Arg Asp Gly Arg Pro Glu Ile Gln Leu
145                 150                 155                 160

His Asn His Trp Ala Gln Leu Thr Val Gly Ala Gly Pro Arg Leu Asp
                165                 170                 175

Asp Gly Arg Trp His Gln Val Glu Val Lys Met Glu Gly Asp Ser Val
            180                 185                 190

Leu Leu Glu Val Asp Gly Glu Glu Val Leu Arg Leu Arg Gln Val Ser
        195                 200                 205

Gly Pro Leu Thr Ser Lys Arg His Pro Ile Met Arg Ile Ala Leu Gly
    210                 215                 220

Gly Leu Leu Phe Pro Ala Ser Asn Leu Arg Leu Pro Leu Val Pro Ala
225                 230                 235                 240

Leu Asp Gly Cys Leu Arg Arg Asp Ser Trp Leu Asp Lys Gln Ala Glu
                245                 250                 255

Ile Ser Ala Ser Ala Pro Thr Ser Leu Arg Ser Cys Asp Val Glu Ser
            260                 265                 270

Asn Pro Gly Ile Phe Leu Pro Pro Gly Thr Gln Ala Glu Phe Asn Leu
        275                 280                 285

Arg Asp Ile Pro Gln Pro His Ala Glu Pro Trp Ala Phe Ser Leu Asp
    290                 295                 300

Leu Gly Leu Lys Gln Ala Ala Gly Ser Gly His Leu Leu Ala Leu Gly
305                 310                 315                 320

Thr Pro Glu Asn Pro Ser Trp Leu Ser Leu His Leu Gln Asp Gln Lys
                325                 330                 335

Val Val Leu Ser Ser Gly Ser
            340

<210>  57
<211>  1089
<212>  DNA
<213>  Artificial

<220>
<223>  Pro_TNFaR1a D41-T127_hinge_SHBG construct

<400>  57
atggatagtg tgtgtcccca aggaaaatat atccaccctc aaaataattc gatttgctgt      60

accaagtgcc acaaaggaac ctacttgtac aatgactgtc caggcccggg gcaggatacg     120

gactgcaggg agtgtgagag cggctccttc accgcttcag aaaaccacct cagacactgc     180

ctcagctgct ccaaatgccg aaaggaaatg ggtcaggtgg agatctcttc ttgcacagtg     240

gaccgggaca ccgtgtgtgg ctgcaggaag aaccagtacc ggcattattg gagtgaaaac     300

cttttccagt gcttcaattg cagcctctgc ctcaatggga ccgtgcacct ctcctgccag     360

gagaaacaga acaccgtgtg cacctgcgac aagacccaca cctgcccccc ctgccccgcc     420

cccgagctgc tgggcggccc ctccgtgttc ctgttccccc ccaagcccaa ggataccaac     480

cctaaggatg actggtttat gctgggactt cgagacggca ggcctgagat ccaactgcac     540

aatcactggg cccagcttac ggtgggtgct ggaccacggc tggatgatgg gagatggcac     600

caggtggaag tcaagatgga gggggactct gtgctgctgg aggtggatgg ggaggaggtg     660

ctgcgcctga gacaggtctc tgggcccctg accagcaaac gccatcccat catgaggatt     720

gcgcttgggg ggctgctctt ccccgcttcc aaccttcggt tgccgctggt tcctgccctg     780

gatggctgcc tgcgccggga ttcctggctg gacaaacagg ccgagatctc agcatctgcc     840

cccactagcc tcagaagctg tgatgtagaa tcaaatcccg ggatatttct ccctccaggg     900

actcaggcag aattcaatct ccgagacatt ccccagcctc atgcagagcc ctgggccttc     960

tctttggacc tgggactcaa gcaggcagca ggctcaggcc acctccttgc tcttgggaca    1020

ccagagaacc catcttggct cagtctccac ctccaagatc aaaaggtggt gttgtcttct    1080

gggtcgtga                                                              1089

<210>  58
<211>  362
<212>  PRT
<213>  Artificial

<220>
<223>  Pro_TNFaR1a D41-T127_hinge_SHBG protein

<400>  58

Met Asp Ser Val Cys Pro Gln Gly Lys Tyr Ile His Pro Gln Asn Asn
1               5                   10                  15

Ser Ile Cys Cys Thr Lys Cys His Lys Gly Thr Tyr Leu Tyr Asn Asp
            20                  25                  30

Cys Pro Gly Pro Gly Gln Asp Thr Asp Cys Arg Glu Cys Glu Ser Gly
        35                  40                  45

Ser Phe Thr Ala Ser Glu Asn His Leu Arg His Cys Leu Ser Cys Ser
    50                  55                  60

Lys Cys Arg Lys Glu Met Gly Gln Val Glu Ile Ser Ser Cys Thr Val
65                  70                  75                  80

Asp Arg Asp Thr Val Cys Gly Cys Arg Lys Asn Gln Tyr Arg His Tyr
                85                  90                  95

Trp Ser Glu Asn Leu Phe Gln Cys Phe Asn Cys Ser Leu Cys Leu Asn
            100                 105                 110

Gly Thr Val His Leu Ser Cys Gln Glu Lys Gln Asn Thr Val Cys Thr
        115                 120                 125

Cys Asp Lys Thr His Thr Cys Pro Pro Cys Pro Ala Pro Glu Leu Leu
    130                 135                 140

Gly Gly Pro Ser Val Phe Leu Phe Pro Pro Lys Pro Lys Asp Thr Asn
145                 150                 155                 160

Pro Lys Asp Asp Trp Phe Met Leu Gly Leu Arg Asp Gly Arg Pro Glu
                165                 170                 175

Ile Gln Leu His Asn His Trp Ala Gln Leu Thr Val Gly Ala Gly Pro
            180                 185                 190

Arg Leu Asp Asp Gly Arg Trp His Gln Val Glu Val Lys Met Glu Gly
        195                 200                 205

Asp Ser Val Leu Leu Glu Val Asp Gly Glu Glu Val Leu Arg Leu Arg
    210                 215                 220

Gln Val Ser Gly Pro Leu Thr Ser Lys Arg His Pro Ile Met Arg Ile
225                 230                 235                 240

Ala Leu Gly Gly Leu Leu Phe Pro Ala Ser Asn Leu Arg Leu Pro Leu
                245                 250                 255

Val Pro Ala Leu Asp Gly Cys Leu Arg Arg Asp Ser Trp Leu Asp Lys
            260                 265                 270

Gln Ala Glu Ile Ser Ala Ser Ala Pro Thr Ser Leu Arg Ser Cys Asp
        275                 280                 285

Val Glu Ser Asn Pro Gly Ile Phe Leu Pro Pro Gly Thr Gln Ala Glu
    290                 295                 300

Phe Asn Leu Arg Asp Ile Pro Gln Pro His Ala Glu Pro Trp Ala Phe
305                 310                 315                 320

Ser Leu Asp Leu Gly Leu Lys Gln Ala Ala Gly Ser Gly His Leu Leu
                325                 330                 335

Ala Leu Gly Thr Pro Glu Asn Pro Ser Trp Leu Ser Leu His Leu Gln
            340                 345                 350

Asp Gln Lys Val Val Leu Ser Ser Gly Ser
        355                 360

<210>  59
<211>  1191
<212>  DNA
<213>  Artificial

<220>

<223>  Pro_TNFaR1a D41-N161_hinge_SHBG construct

<400>  59
atggatagtg tgtgtcccca aggaaaatat atccaccctc aaaataattc gatttgctgt      60

accaagtgcc acaaaggaac ctacttgtac aatgactgtc caggcccggg gcaggatacg     120

gactgcaggg agtgtgagag cggctccttc accgcttcag aaaaccacct cagacactgc     180

ctcagctgct ccaaatgccg aaaggaaatg ggtcaggtgg agatctcttc ttgcacagtg     240

gaccgggaca ccgtgtgtgg ctgcaggaag aaccagtacc ggcattattg gagtgaaaac     300

cttttccagt gcttcaattg cagcctctgc ctcaatggga ccgtgcacct ctcctgccag     360

gagaaacaga acaccgtgtg cacctgccat gcaggtttct ttctaagaga aaacgagtgt     420

gtctcctgta gtaactgtaa gaaaagcctg gagtgcacga agttgtgcct accccagatt     480

gagaattgcg acaagaccca cacctgcccc ccctgccccg cccccgagct gctgggcggc     540

ccctccgtgt tcctgttccc ccccaagccc aaggatacca accctaagga tgactggttt     600

atgctgggac ttcgagacgg caggcctgag atccaactgc acaatcactg ggcccagctt     660

acggtgggtg ctggaccacg gctggatgat gggagatggc accaggtgga agtcaagatg     720

gagggggact ctgtgctgct ggaggtggat ggggaggagg tgctgcgcct gagacaggtc     780

tctgggcccc tgaccagcaa acgccatccc atcatgagga ttgcgcttgg ggggctgctc     840

ttccccgctt ccaaccttcg gttgccgctg gttcctgccc tggatggctg cctgcgccgg     900

gattcctggc tggacaaaca ggccgagatc tcagcatctg cccccactag cctcagaagc     960

tgtgatgtag aatcaaatcc cgggatattt ctccctccag ggactcaggc agaattcaat    1020

ctccgagaca ttccccagcc tcatgcagag ccctgggcct tctctttgga cctgggactc    1080

aagcaggcag caggctcagg ccacctcctt gctcttggga caccagagaa cccatcttgg    1140

ctcagtctcc acctccaaga tcaaaaggtg gtgttgtctt ctgggtcgtg a             1191


<210>  60
<211>  396
<212>  PRT
<213>  Artificial

<220>
<223>  Pro_TNFaR1a D41-N161_hinge_SHBG protein

<400>  60

Met Asp Ser Val Cys Pro Gln Gly Lys Tyr Ile His Pro Gln Asn Asn
1               5                   10                  15

Ser Ile Cys Cys Thr Lys Cys His Lys Gly Thr Tyr Leu Tyr Asn Asp
            20                  25                  30

Cys Pro Gly Pro Gly Gln Asp Thr Asp Cys Arg Glu Cys Glu Ser Gly
        35                  40                  45

Ser Phe Thr Ala Ser Glu Asn His Leu Arg His Cys Leu Ser Cys Ser
    50                  55                  60

Lys Cys Arg Lys Glu Met Gly Gln Val Glu Ile Ser Ser Cys Thr Val
65                  70                  75                  80

Asp Arg Asp Thr Val Cys Gly Cys Arg Lys Asn Gln Tyr Arg His Tyr
                85                  90                  95

Trp Ser Glu Asn Leu Phe Gln Cys Phe Asn Cys Ser Leu Cys Leu Asn
            100                 105                 110

Gly Thr Val His Leu Ser Cys Gln Glu Lys Gln Asn Thr Val Cys Thr
        115                 120                 125

Cys His Ala Gly Phe Phe Leu Arg Glu Asn Glu Cys Val Ser Cys Ser
    130                 135                 140

Asn Cys Lys Lys Ser Leu Glu Cys Thr Lys Leu Cys Leu Pro Gln Ile
145                 150                 155                 160

Glu Asn Cys Asp Lys Thr His Thr Cys Pro Pro Cys Pro Ala Pro Glu
                165                 170                 175

Leu Leu Gly Gly Pro Ser Val Phe Leu Phe Pro Pro Lys Pro Lys Asp
            180                 185                 190

Thr Asn Pro Lys Asp Asp Trp Phe Met Leu Gly Leu Arg Asp Gly Arg
        195                 200                 205

Pro Glu Ile Gln Leu His Asn His Trp Ala Gln Leu Thr Val Gly Ala
    210                 215                 220

Gly Pro Arg Leu Asp Asp Gly Arg Trp His Gln Val Glu Val Lys Met
225                 230                 235                 240

Glu Gly Asp Ser Val Leu Leu Glu Val Asp Gly Glu Glu Val Leu Arg
                245                 250                 255

Leu Arg Gln Val Ser Gly Pro Leu Thr Ser Lys Arg His Pro Ile Met
            260                 265                 270

Arg Ile Ala Leu Gly Gly Leu Leu Phe Pro Ala Ser Asn Leu Arg Leu
        275                 280                 285

Pro Leu Val Pro Ala Leu Asp Gly Cys Leu Arg Arg Asp Ser Trp Leu
    290                 295                 300

Asp Lys Gln Ala Glu Ile Ser Ala Ser Ala Pro Thr Ser Leu Arg Ser
305                 310                 315                 320

Cys Asp Val Glu Ser Asn Pro Gly Ile Phe Leu Pro Pro Gly Thr Gln
                325                 330                 335

Ala Glu Phe Asn Leu Arg Asp Ile Pro Gln Pro His Ala Glu Pro Trp
            340                 345                 350

Ala Phe Ser Leu Asp Leu Gly Leu Lys Gln Ala Ala Gly Ser Gly His
        355                 360                 365

Leu Leu Ala Leu Gly Thr Pro Glu Asn Pro Ser Trp Leu Ser Leu His
    370                 375                 380

Leu Gln Asp Gln Lys Val Val Leu Ser Ser Gly Ser
385                 390                 395

<210>  61
<211>  1149
<212>  DNA
<213>  Artificial

<220>
<223>  Euk_TNFaR1a M1-L108_hinge_SHBG construct

<400>  61
atgggcctct ccaccgtgcc tgacctgctg ctgccactgg tgctcctgga gctgttggtg      60

ggaatatacc cctcaggggt tattggactg gtccctcacc taggggacag ggagaagaga     120

gatagtgtgt gtccccaagg aaaatatatc caccctcaaa ataattcgat ttgctgtacc     180

aagtgccaca aaggaaccta cttgtacaat gactgtccag gcccggggca ggatacggac 240

tgcagggagt gtgagagcgg ctccttcacc gcttcagaaa accacctcag acactgcctc 300

agctgctcca aatgccgaaa ggaaatgggt caggtggaga tctcttcttg cacagtggac 360

cgggacaccg tgtgtggctg caggaagaac cagtaccggc attattggag tgaaaacctt 420

ttccagtgct tcaattgcag cctctgcgac aagacccaca cctgcccccc ctgccccgcc 480

cccgagctgc tgggcggccc ctccgtgttc ctgttccccc ccaagcccaa ggataccaac 540

cctaaggatg actggtttat gctgggactt cgagacggca ggcctgagat ccaactgcac 600

aatcactggg cccagcttac ggtgggtgct ggaccacggc tggatgatgg gagatggcac 660

caggtggaag tcaagatgga gggggactct gtgctgctgg aggtggatgg ggaggaggtg 720

ctgcgcctga gacaggtctc tgggcccctg accagcaaac gccatcccat catgaggatt 780

gcgcttgggg ggctgctctt ccccgcttcc aaccttcggt tgccgctggt tcctgccctg 840

gatggctgcc tgcgccggga ttcctggctg gacaaacagg ccgagatctc agcatctgcc 900

cccactagcc tcagaagctg tgatgtagaa tcaaatcccg ggatatttct ccctccaggg 960

actcaggcag aattcaatct ccgagacatt ccccagcctc atgcagagcc ctgggccttc 1020

tctttggacc tgggactcaa gcaggcagca ggctcaggcc acctccttgc tcttgggaca 1080

ccagagaacc catcttggct cagtctccac ctccaagatc aaaaggtggt gttgtcttct 1140

gggtcgtga 1149

<210> 62
<211> 382
<212> PRT
<213> Artificial

<220>
<223> Euk_TNFaR1a M1-L108_hinge_SHBG protein

<400> 62

Met Gly Leu Ser Thr Val Pro Asp Leu Leu Leu Pro Leu Val Leu Leu
1               5                   10                  15

Glu Leu Leu Val Gly Ile Tyr Pro Ser Gly Val Ile Gly Leu Val Pro
            20                  25                  30

His Leu Gly Asp Arg Glu Lys Arg Asp Ser Val Cys Pro Gln Gly Lys
        35                  40                  45

Tyr Ile His Pro Gln Asn Asn Ser Ile Cys Cys Thr Lys Cys His Lys
    50                  55                  60

Gly Thr Tyr Leu Tyr Asn Asp Cys Pro Gly Pro Gly Gln Asp Thr Asp
65                  70                  75                  80

Cys Arg Glu Cys Glu Ser Gly Ser Phe Thr Ala Ser Glu Asn His Leu
                85                  90                  95

Arg His Cys Leu Ser Cys Ser Lys Cys Arg Lys Glu Met Gly Gln Val
            100                 105                 110

Glu Ile Ser Ser Cys Thr Val Asp Arg Asp Thr Val Cys Gly Cys Arg
        115                 120                 125

Lys Asn Gln Tyr Arg His Tyr Trp Ser Glu Asn Leu Phe Gln Cys Phe
    130                 135                 140

Asn Cys Ser Leu Cys Asp Lys Thr His Thr Cys Pro Pro Cys Pro Ala
145                 150                 155                 160

Pro Glu Leu Leu Gly Gly Pro Ser Val Phe Leu Phe Pro Pro Lys Pro
                165                 170                 175

Lys Asp Thr Asn Pro Lys Asp Asp Trp Phe Met Leu Gly Leu Arg Asp
            180                 185                 190

Gly Arg Pro Glu Ile Gln Leu His Asn His Trp Ala Gln Leu Thr Val
        195                 200                 205

Gly Ala Gly Pro Arg Leu Asp Asp Gly Arg Trp His Gln Val Glu Val
    210                 215                 220

Lys Met Glu Gly Asp Ser Val Leu Leu Glu Val Asp Gly Glu Glu Val
225                 230                 235                 240

Leu Arg Leu Arg Gln Val Ser Gly Pro Leu Thr Ser Lys Arg His Pro
                245                 250                 255

Ile Met Arg Ile Ala Leu Gly Gly Leu Leu Phe Pro Ala Ser Asn Leu
            260                 265                 270

Arg Leu Pro Leu Val Pro Ala Leu Asp Gly Cys Leu Arg Arg Asp Ser
        275                 280                 285

Trp Leu Asp Lys Gln Ala Glu Ile Ser Ala Ser Ala Pro Thr Ser Leu
    290                 295                 300

Arg Ser Cys Asp Val Glu Ser Asn Pro Gly Ile Phe Leu Pro Pro Gly
305                 310                 315                 320

Thr Gln Ala Glu Phe Asn Leu Arg Asp Ile Pro Gln Pro His Ala Glu
                325                 330                 335

Pro Trp Ala Phe Ser Leu Asp Leu Gly Leu Lys Gln Ala Ala Gly Ser
            340                 345                 350

Gly His Leu Leu Ala Leu Gly Thr Pro Glu Asn Pro Ser Trp Leu Ser
        355                 360                 365

Leu His Leu Gln Asp Gln Lys Val Val Leu Ser Ser Gly Ser
    370                 375                 380

<210>  63
<211>  1206
<212>  DNA
<213>  Artificial

<220>
<223>  Euk_TNFaR1a M1-T127_hinge_SHBG construct

<400>  63
atgggcctct ccaccgtgcc tgacctgctg ctgccactgg tgctcctgga gctgttggtg      60

ggaatatacc cctcaggggt tattggactg gtccctcacc taggggacag ggagaagaga     120

gatagtgtgt gtccccaagg aaaatatatc caccctcaaa ataattcgat ttgctgtacc     180

aagtgccaca aaggaaccta cttgtacaat gactgtccag gcccggggca ggatacggac     240

tgcagggagt gtgagagcgg ctccttcacc gcttcagaaa accacctcag acactgcctc     300

agctgctcca aatgccgaaa ggaaatgggt caggtggaga tctcttcttg cacagtggac     360

cgggacaccg tgtgtggctg caggaagaac cagtaccggc attattggag tgaaaacctt     420

ttccagtgct tcaattgcag cctctgcctc aatgggaccg tgcacctctc ctgccaggag     480

aaacagaaca ccgtgtgcac ctgcgacaag acccacacct gccccccctg ccccgccccc     540

gagctgctgg gcggcccctc cgtgttcctg ttccccccca agcccaagga taccaaccct    600

aaggatgact ggtttatgct gggacttcga gacggcaggc ctgagatcca actgcacaat    660

cactgggccc agcttacggt gggtgctgga ccacggctgg atgatgggag atggcaccag    720

gtggaagtca agatggaggg ggactctgtg ctgctggagg tggatgggga ggaggtgctg    780

cgcctgagac aggtctctgg gcccctgacc agcaaacgcc atcccatcat gaggattgcg    840

cttggggggc tgctcttccc cgcttccaac cttcggttgc cgctggttcc tgccctggat    900

ggctgcctgc gccgggattc ctggctggac aaacaggccg agatctcagc atctgccccc    960

actagcctca gaagctgtga tgtagaatca aatcccggga tatttctccc tccagggact   1020

caggcagaat tcaatctccg agacattccc cagcctcatg cagagccctg ggccttctct   1080

ttggacctgg gactcaagca ggcagcaggc tcaggccacc tccttgctct tgggacacca   1140

gagaacccat cttggctcag tctccacctc caagatcaaa aggtggtgtt gtcttctggg   1200

tcgtga                                                              1206

<210>  64
<211>  401
<212>  PRT
<213>  Artificial

<220>
<223>  Euk_TNFaR1a M1-T127_hinge_SHBG  protein

<400>  64

Met Gly Leu Ser Thr Val Pro Asp Leu Leu Leu Pro Leu Val Leu Leu
1               5                   10                  15

Glu Leu Leu Val Gly Ile Tyr Pro Ser Gly Val Ile Gly Leu Val Pro
            20                  25                  30

His Leu Gly Asp Arg Glu Lys Arg Asp Ser Val Cys Pro Gln Gly Lys
        35                  40                  45

Tyr Ile His Pro Gln Asn Asn Ser Ile Cys Cys Thr Lys Cys His Lys
    50                  55                  60

Gly Thr Tyr Leu Tyr Asn Asp Cys Pro Gly Pro Gly Gln Asp Thr Asp
65                  70                  75                  80

Cys Arg Glu Cys Glu Ser Gly Ser Phe Thr Ala Ser Glu Asn His Leu
                85                  90                  95

Arg His Cys Leu Ser Cys Ser Lys Cys Arg Lys Glu Met Gly Gln Val
            100                 105                 110

Glu Ile Ser Ser Cys Thr Val Asp Arg Asp Thr Val Cys Gly Cys Arg
        115                 120                 125

Lys Asn Gln Tyr Arg His Tyr Trp Ser Glu Asn Leu Phe Gln Cys Phe
    130                 135                 140

Asn Cys Ser Leu Cys Leu Asn Gly Thr Val His Leu Ser Cys Gln Glu
145                 150                 155                 160

Lys Gln Asn Thr Val Cys Thr Cys Asp Lys Thr His Thr Cys Pro Pro
                165                 170                 175

Cys Pro Ala Pro Glu Leu Leu Gly Gly Pro Ser Val Phe Leu Phe Pro
            180                 185                 190

Pro Lys Pro Lys Asp Thr Asn Pro Lys Asp Asp Trp Phe Met Leu Gly
        195                 200                 205

Leu Arg Asp Gly Arg Pro Glu Ile Gln Leu His Asn His Trp Ala Gln
    210                 215                 220

Leu Thr Val Gly Ala Gly Pro Arg Leu Asp Asp Gly Arg Trp His Gln
225                 230                 235                 240

Val Glu Val Lys Met Glu Gly Asp Ser Val Leu Leu Glu Val Asp Gly
                245                 250                 255

Glu Glu Val Leu Arg Leu Arg Gln Val Ser Gly Pro Leu Thr Ser Lys
            260                 265                 270

Arg His Pro Ile Met Arg Ile Ala Leu Gly Gly Leu Leu Phe Pro Ala
        275                 280                 285

Ser Asn Leu Arg Leu Pro Leu Val Pro Ala Leu Asp Gly Cys Leu Arg
    290                 295                 300

Arg Asp Ser Trp Leu Asp Lys Gln Ala Glu Ile Ser Ala Ser Ala Pro
305                 310                 315                 320

Thr Ser Leu Arg Ser Cys Asp Val Glu Ser Asn Pro Gly Ile Phe Leu
                325                 330                 335

Pro Pro Gly Thr Gln Ala Glu Phe Asn Leu Arg Asp Ile Pro Gln Pro
            340                 345                 350

His Ala Glu Pro Trp Ala Phe Ser Leu Asp Leu Gly Leu Lys Gln Ala
        355                 360                 365

Ala Gly Ser Gly His Leu Leu Ala Leu Gly Thr Pro Glu Asn Pro Ser
    370                 375                 380

Trp Leu Ser Leu His Leu Gln Asp Gln Lys Val Val Leu Ser Ser Gly
385                 390                 395                 400

Ser

<210>  65
<211>  1308
<212>  DNA
<213>  Artificial

<220>
<223>  Euk_TNFaR1a M1-N161_hinge_SHBG construct

<400>  65
atgggcctct ccaccgtgcc tgacctgctg ctgccactgg tgctcctgga gctgttggtg      60

ggaatatacc cctcaggggt tattggactg gtccctcacc taggggacag ggagaagaga     120

gatagtgtgt gtccccaagg aaaatatatc caccctcaaa ataattcgat ttgctgtacc     180

aagtgccaca aaggaaccta cttgtacaat gactgtccag gcccggggca ggatacggac     240

tgcagggagt gtgagagcgg ctccttcacc gcttcagaaa accacctcag acactgcctc     300

agctgctcca aatgccgaaa ggaaatgggt caggtggaga tctcttcttg cacagtggac     360

cgggacaccg tgtgtggctg caggaagaac cagtaccggc attattggag tgaaaacctt     420

ttccagtgct tcaattgcag cctctgcctc aatgggaccg tgcacctctc ctgccaggag     480

aaacagaaca ccgtgtgcac ctgccatgca ggtttctttc taagagaaaa cgagtgtgtc     540

tcctgtagta actgtaagaa aagcctggag tgcacgaagt tgtgcctacc ccagattgag     600

aattgcgaca agacccacac ctgccccccc tgccccgccc ccgagctgct gggcggcccc 660

tccgtgttcc tgttcccccc caagcccaag gataccaacc ctaaggatga ctggtttatg 720

ctgggacttc gagacggcag gcctgagatc caactgcaca atcactgggc ccagcttacg 780

gtgggtgctg gaccacggct ggatgatggg agatggcacc aggtggaagt caagatggag 840

ggggactctg tgctgctgga ggtggatggg gaggaggtgc tgcgcctgag acaggtctct 900

gggcccctga ccagcaaacg ccatcccatc atgaggattg cgcttggggg gctgctcttc 960

cccgcttcca accttcggtt gccgctggtt cctgccctgg atggctgcct gcgccgggat 1020

tcctggctgg acaaacaggc cgagatctca gcatctgccc ccactagcct cagaagctgt 1080

gatgtagaat caaatcccgg gatatttctc cctccaggga ctcaggcaga attcaatctc 1140

cgagacattc cccagcctca tgcagagccc tgggccttct ctttggacct gggactcaag 1200

caggcagcag gctcaggcca cctccttgct cttgggacac cagagaaccc atcttggctc 1260

agtctccacc tccaagatca aaaggtggtg ttgtcttctg ggtcgtga 1308

<210> 66
<211> 435
<212> PRT
<213> Artificial

<220>
<223> Euk_TNFaR1a M1-N161_hinge_SHBG protein

<400> 66

Met Gly Leu Ser Thr Val Pro Asp Leu Leu Leu Pro Leu Val Leu Leu
1               5                   10                  15

Glu Leu Leu Val Gly Ile Tyr Pro Ser Gly Val Ile Gly Leu Val Pro
            20                  25                  30

His Leu Gly Asp Arg Glu Lys Arg Asp Ser Val Cys Pro Gln Gly Lys
        35                  40                  45

Tyr Ile His Pro Gln Asn Asn Ser Ile Cys Cys Thr Lys Cys His Lys
    50                  55                  60

Gly Thr Tyr Leu Tyr Asn Asp Cys Pro Gly Pro Gly Gln Asp Thr Asp
65                  70                  75                  80

Cys Arg Glu Cys Glu Ser Gly Ser Phe Thr Ala Ser Glu Asn His Leu
                85                  90                  95

Arg His Cys Leu Ser Cys Ser Lys Cys Arg Lys Glu Met Gly Gln Val
            100                 105                 110

Glu Ile Ser Ser Cys Thr Val Asp Arg Asp Thr Val Cys Gly Cys Arg
        115                 120                 125

Lys Asn Gln Tyr Arg His Tyr Trp Ser Glu Asn Leu Phe Gln Cys Phe
    130                 135                 140

Asn Cys Ser Leu Cys Leu Asn Gly Thr Val His Leu Ser Cys Gln Glu
145                 150                 155                 160

Lys Gln Asn Thr Val Cys Thr Cys His Ala Gly Phe Phe Leu Arg Glu
                165                 170                 175

Asn Glu Cys Val Ser Cys Ser Asn Cys Lys Lys Ser Leu Glu Cys Thr
            180                 185                 190

Lys Leu Cys Leu Pro Gln Ile Glu Asn Cys Asp Lys Thr His Thr Cys
        195                 200                 205

Pro Pro Cys Pro Ala Pro Glu Leu Leu Gly Gly Pro Ser Val Phe Leu
    210                 215                 220

Phe Pro Pro Lys Pro Lys Asp Thr Asn Pro Lys Asp Asp Trp Phe Met
225                 230                 235                 240

Leu Gly Leu Arg Asp Gly Arg Pro Glu Ile Gln Leu His Asn His Trp
                245                 250                 255

Ala Gln Leu Thr Val Gly Ala Gly Pro Arg Leu Asp Asp Gly Arg Trp
            260                 265                 270

His Gln Val Glu Val Lys Met Glu Gly Asp Ser Val Leu Leu Glu Val
        275                 280                 285

Asp Gly Glu Glu Val Leu Arg Leu Arg Gln Val Ser Gly Pro Leu Thr
    290                 295                 300

Ser Lys Arg His Pro Ile Met Arg Ile Ala Leu Gly Gly Leu Leu Phe
305                 310                 315                 320

Pro Ala Ser Asn Leu Arg Leu Pro Leu Val Pro Ala Leu Asp Gly Cys
                325                 330                 335

Leu Arg Arg Asp Ser Trp Leu Asp Lys Gln Ala Glu Ile Ser Ala Ser
            340                 345                 350

Ala Pro Thr Ser Leu Arg Ser Cys Asp Val Glu Ser Asn Pro Gly Ile
        355                 360                 365

Phe Leu Pro Pro Gly Thr Gln Ala Glu Phe Asn Leu Arg Asp Ile Pro
    370                 375                 380

Gln Pro His Ala Glu Pro Trp Ala Phe Ser Leu Asp Leu Gly Leu Lys
385                 390                 395                 400

Gln Ala Ala Gly Ser Gly His Leu Leu Ala Leu Gly Thr Pro Glu Asn
                405                 410                 415

Pro Ser Trp Leu Ser Leu His Leu Gln Asp Gln Lys Val Val Leu Ser
            420                 425                 430

Ser Gly Ser
        435

<210>  67
<211>  716
<212>  DNA
<213>  Artificial

<220>
<223>  Ig hinge SHBG construct

<400>  67
atgtgcgaca agacccacac ctgccccccc tgccccgccc ccgagctgct gggcggcccc      60

tccgtgttcc tgttcccccc caagcccaag gataccaacc ctaaggatga ctggtttatg     120

ctgggacttc gagacggcag gcctgagatc caactgcaca atcactgggc ccagcttacg     180

gtgggtgctg gaccacggct ggatgatggg agatggcacc aggtggaagt caagatggag     240

ggggactctg tgctgctgga ggtggatggg gaggaggtgc tgcgcctgag acaggtctct     300

gggcccctga ccagcaaacg ccatcccatc atgaggattg cgcttggggg gctgctcttc     360

cccgcttcca accttcggtt gccgctggtt cctgccctgg atggctgcct gcgccgggat 420

tcctggctgg acaaacaggc cgagatctca gcatctgccc ccactagcct cagaagctgt 480

gatgtagaat caaatcccgg gatatttctc cctccaggga ctcaggcaga attcaatctc 540

cgagacattc cccagcctca tgcagagccc tgggccttct ctttggacct gggactcaag 600

caggcagcag gctcaggcca cctccttgct cttgggacac cagagaaccc atcttggctc 660

agtctccacc tccaagatca aaaggtggtg ttgtcttctg ggtcgtgagg atccaa 716

<210> 68
<211> 18
<212> DNA
<213> Artificial

<220>
<223> Oligonucleotide primer

<400> 68
aacatatgcc cgcccagg 18

<210> 69
<211> 33
<212> DNA
<213> Artificial

<220>
<223> Oligonucleotide primer

<400> 69
cccggctggt actgctgcga caagacccac acc 33

<210> 70
<211> 33
<212> DNA
<213> Artificial

<220>
<223> Oligonucleotide primer

<400> 70
ggtgtgggtc ttgtcgcagc agtaccagcc ggg 33

<210> 71
<211> 35
<212> DNA
<213> Artificial

<220>

<223>  Oligonucleotide primer

<400>  71
gttcaatggt cgaaccattt atcatcgtgt ttttc                                35


<210>  72
<211>  35
<212>  DNA
<213>  Artificial

<220>
<223>  Oligonucleotide primer

<400>  72
gaaaaacacg atgataaatg gttcgaccat tgaac                                35


<210>  73
<211>  20
<212>  DNA
<213>  Artificial

<220>
<223>  Oligonucleotide primer

<400>  73
gctagccgcc cctctccctc                                                 20


<210>  74
<211>  20
<212>  DNA
<213>  Artificial

<220>
<223>  Oligonucleotide primer

<400>  74
ggatccttag tctttcttct                                                 20


<210>  75
<211>  1152
<212>  DNA
<213>  Artificial

<220>
<223>  IRES mouse DHFR sequence

<400>  75
gctagccgcc cctctccctc ccccccccct aacgttactg gccgaagccg cttggaataa      60

ggccggtgtg cgtttgtcta tatgttattt tccaccatat tgccgtcttt tggcaatgtg    120

agggcccgga aacctggccc tgtcttcttg acgagcattc ctaggggtct ttcccctctc    180

gccaaaggaa tgcaaggtct gttgaatgtc gtgaaggaag cagttcctct ggaagcttct 240

tgaagacaaa caacgtctgt agcgaccctt tgcaggcagc ggaacccccc acctggcgac 300

aggtgcctct gcggccaaaa gccacgtgta taagatacac ctgcaaaggc ggcacaaccc 360

cagtgccacg ttgtgagttg gatagttgtg gaaagagtca aatggctctc ctcaagcgta 420

ttcaacaagg ggctgaagga tgcccagaag gtaccccatt gtatgggatc tgatctgggg 480

cctcggtgca catgctttac atgtgtttag tcgaggttaa aaaaacgtct aggccccccg 540

aaccacgggg acgtggtttt cctttgaaaa acacgatgat aaatggttcg accattgaac 600

tgcatcgtcg ccgtgtccca aaatatgggg attggcaaga acggagacct accctggcct 660

ccgctcagga acgagttcaa gtacttccaa agaatgacca caacctcttc agtggaaggt 720

aaacagaatc tggtgattat gggtaggaaa acctggttct ccattcctga gaagaatcga 780

cctttaaagg acagaattaa tatagttctc agtagagaac tcaaagaacc accacgagga 840

gctcattttc ttgccaaaag tttggatgat gccttaagac ttattgaaca accggaattg 900

gcaagtaaag tagacatggt ttggatagtc ggaggcagtt ctgtttacca ggaagccatg 960

aatcaaccag gccacctcag actctttgtg acaaggatca tgcaggaatt tgaaagtgac 1020

acgtttttcc cagaaattga tttggggaaa tataaacttc tcccagaata cccaggcgtc 1080

ctctctgagg tccaggagga aaaaggcatc aagtataagt ttgaagtcta cgagaagaaa 1140

gactaaggat cc 1152

<210> 76
<211> 3342
<212> DNA
<213> Artificial

<220>
<223> pCDSRalpha 296 MCSII IRES mouse DHFR sequence

<400> 76
gtcgacctca tgtttgacag cttatcatcg ataagcttct gtggaatgtg tgtcagttag 60

ggtgtggaaa gtccccaggc tccccagcag gcagaagtat gcaaagcatg catctcaatt 120

agtcagcaac caggtgtgga aagtccccag gctccccagc aggcagaagt atgcaaagca 180

tgcatctcaa ttagtcagca accatagtcc cgcccctaac tccgcccatc ccgcccctaa 240

ctccgcccag ttccgcccat tctccgcccc atggctgact aatttttttt atttatgcag 300

aggccgaggc cgcctcggcc tctgagctat tccagaagta gtgaggaggc ttttttggag 360

gcctaggctt ttgcaaaaag ctcctccgat cgaggggctc gcatctctcc ttcacgcgcc 420

cgccgcccta cctgaggccg ccatccacgc cggttgagtc gcgttctgcc gcctcccgcc 480

tgtggtgcct cctgaactgc gtccgccgtc taggtaagtt taaagctcag gtcgagaccg 540

ggcctttgtc cggcgctccc ttggagccta cctagactca gcccggctct ccacgctttg 600

cctgaccctg cttgctcaac tctacgtctt tgtttcgttt ttgttctgcg ccgttacaga 660

tccaagctgc ctcgaggaac tgaaaaacca gaaagttaac tggtaagttt agtctttttg 720

tcttttattt caggtcccgg atctggtacc gagctcacta gtacgcgtga attctgcaga 780

tatccagctg gcggccgcgc tagcgggccc ggatccagac atgataagat acattgatga 840

gtttggacaa accacaacta gaatgcagtg aaaaaaatgc tttatttgtg aaatttgtga 900

tgctattgct ttatttgtaa ccattataag ctgcaataaa caagttaaca acaacaattg 960

cattcatttt atgtttcagg ttcaggggga ggtgtgggag gttttttaaa gcaagtaaaa 1020

cctctacaaa tgtggtatgg ctgattatga tcctgcctcg cgcgtttcgg tgatgacggt 1080

gaaaacctct gacacatgca gctcccggag acggtcacag cttgtctgta agcggatgcc 1140

gggagcagac aagcccgtca gggcgcgtca gcgggtgttg gcgggtgtcg gggcgcagcc 1200

atgacccagt cacgtagcga tagcggagtg tatactggct taactatgcg gcatcagagc 1260

agattgtact gagagtgcac catatgcggt gtgaaatacc gcacagatgc gtaaggagaa 1320

aataccgcat caggcgctct tccgcttcct cgctcactga ctcgctgcgc tcggtcgttc 1380

ggctgcggcg agcggtatca gctcactcaa aggcggtaat acggttatcc acagaatcag 1440

gggataacgc aggaaagaac atgtgagcaa aaggccagca aaaggccagg aaccgtaaaa 1500

aggccgcgtt gctggcgttt ttccataggc tccgcccccc tgacgagcat cacaaaaatc 1560

gacgctcaag tcagaggtgg cgaaacccga caggactata aagataccag gcgtttcccc 1620

ctggaagctc cctcgtgcgc tctcctgttc cgaccctgcc gcttaccgga tacctgtccg 1680

cctttctccc ttcgggaagc gtggcgcttt ctcaatgctc acgctgtagg tatctcagtt 1740

cggtgtaggt cgttcgctcc aagctgggct gtgtgcacga accccccgtt cagcccgacc 1800

gctgcgcctt atccggtaac tatcgtcttg agtccaaccc ggtaagacac gacttatcgc 1860

cactggcagc agccactggt aacaggatta gcagagcgag gtatgtaggc ggtgctacag 1920

agttcttgaa gtggtggcct aactacggct acactagaag gacagtattt ggtatctgcg 1980

ctctgctgaa gccagttacc ttcggaaaaa gagttggtag ctcttgatcc ggcaaacaaa 2040

ccaccgctgg tagcggtggt ttttttgttt gcaagcagca gattacgcgc agaaaaaaag 2100

gatctcaaga agatcctttg atcttttcta cggggtctga cgctcagtgg aacgaaaact 2160

cacgttaagg gattttggtc atgagattat caaaaaggat cttcacctag atccttttaa 2220

attaaaaatg aagttttaaa tcaatctaaa gtatatatga gtaaacttgg tctgacagtt 2280

accaatgctt aatcagtgag gcacctatct cagcgatgtg tctatttcgt tcatccatag 2340

ttgcctgact ccccgtcgtg tagataacta cgatacggga gggcttacca tctggcccca 2400

gtgctgcaat gataccgcga gacccacgct caccggctcc agatttatca gcaataaacc 2460

agccagccgg aagggccgag cgcagaagtg gtcctgcaac tttatccgcc tccatccagt 2520

ctattaattg ttgccgggaa gctagagtaa gtagttcgcc agttaatagt ttgcgcaacg 2580

ttgttgccat tgctacaggc atcgtggtgt cacgctcgtc gtttggtatg gcttcattca 2640

gctccggttc ccaacgatca aggcgagtta catgatcccc catgttgtgc aaaaaagcgg 2700

ttagctcctt cggtcctccg atcgttgtca gaagtaagtt ggccgcagtg ttatcactca 2760

tggttatggc agcactgcat aattctctta ctgtcatgcc atccgtaaca tgcttttctg 2820

tgactggtga gtactcaacc aagtcattct gagaatagtg tatgcggcga ccgagttgct 2880

cttgcccggc gtcaatacgg gataataccg cgccacatag cagaacttta aaagtgctca 2940

tcattggaaa acgttcttcg gggcgaaaac tctcaaggat cttaccgctg ttgagatcca 3000

gttcgatgta acccactcgt gcacccaact gatcttcagc atcttttact ttcaccagcg 3060

tttctgggtg agcaaaaaca ggaaggcaaa atgccgcaaa aaagggaata agggcgacac 3120

ggaaatgttg aatactcatc ttcctttttc aatattattg aagcatttat cagggttatt 3180

gtctcatgag cggatacata tttgaatgta tttagaaaaa taaacaaata ggggttccgc 3240

gcacatttcc ccgaaaagtg ccacctgacg tctaagaaac cattattatc atgacattaa 3300

cctataaaaa taggcgtatc acgaggccct ttcgtcttca ag 3342

<210> 77
<211> 30
<212> DNA
<213> Artificial

<220>
<223> Oligonucleotide primer

<400> 77
atcccctgac aagctgccag gcaggttctc 30

<210>  78
<211>  30
<212>  DNA
<213>  Artificial

<220>
<223>  Oligonucleotide primer

<400>  78
aggggaggcg tttgggaagg ttggatgttc                                        30


<210>  79
<211>  823
<212>  DNA
<213>  Homo sapiens

<400>  79
atcccctgac aagctgccag gcaggttctc ttcctctcac atactgaccc acggctccac      60

cctctctccc ctggaaagga caccatgagc actgaaagca tgatccggga cgtggagctg     120

gccgaggagg cgctccccaa gaagacaggg gggccccagg gctccaggcg gtgcttgttc     180

ctcagcctct tctccttcct gatcgtggca ggcgccacca cgctcttctg cctgctgcac     240

tttggagtga tcggccccca gagggaagag ttccccaggg acctctctct aatcagccct     300

ctggcccagg cagtcagatc atcttctcga accccgagtg acaagcctgt agcccatgtt     360

gtagcaaacc ctcaagctga ggggcagctc cagtggctga accgccgggc caatgccctc     420

ctggccaatg gcgtggagct gagagataac cagctggtgg tgccatcaga gggcctgtac     480

ctcatctact cccaggtcct cttcaagggc caaggctgcc cctccaccca tgtgctcctc     540

acccacacca tcagccgcat cgccgtctcc taccagacca aggtcaacct cctctctgcc     600

atcaagagcc cctgccagag ggagacccca gagggggctg aggccaagcc ctggtatgag     660

cccatctatc tgggaggggt cttccagctg gagaagggtg accgactcag cgctgagatc     720

aatcggcccg actatctcga ctttgccgag tctgggcagg tctactttgg gatcattgcc     780

ctgtgaggag gacgaacatc caaccttccc aaacgcctcc cct                       823


<210>  80
<211>  474
<212>  DNA
<213>  Homo sapiens

<400>  80
gtcagatcat cttctcgaac cccgagtgac aagcctgtag cccatgttgt agcaaaccct      60

caagctgagg ggcagctcca gtggctgaac cgccgggcca atgccctcct ggccaatggc    120

gtggagctga gagataacca gctggtggtg ccatcagagg gcctgtacct catctactcc    180

caggtcctct tcaagggcca aggctgcccc tccacccatg tgctcctcac ccacaccatc    240

agccgcatcg ccgtctccta ccagaccaag gtcaacctcc tctctgccat caagagcccc    300

tgccagaggg agaccccaga gggggctgag gccaagccct ggtatgagcc catctatctg    360

ggaggggtct tccagctgga gaagggtgac cgactcagcg ctgagatcaa tcggcccgac    420

tatctcgact ttgccgagtc tgggcaggtc tactttggga tcattgccct gtga          474

<210>  81
<211>  29
<212>  DNA
<213>  Artificial

<220>
<223>  Oligonucleotide primer

<400>  81
aaccatatgg tcagatcatc ttctcgaac                                        29

<210>  82
<211>  23
<212>  DNA
<213>  Artificial

<220>
<223>  Oligonucleotide primer

<400>  82
ggttggatcc tcacagggca atg                                              23

<210>  83
<211>  24
<212>  DNA
<213>  Artificial

<220>
<223>  Oligonucleotide primer

<400>  83
ttggatcctt aacagatctg gtgg                                             24

<210>  84
<211>  492
<212>  DNA
<213>  Homo sapiens

<400>  84
atgcccgccc aggtggcatt tacaccctac gccccggagc ccgggagcac atgccggctc      60

agagaatact atgaccagac agctcagatg tgctgcagca aatgctcgcc gggccaacat     120

gcaaaagtct tctgtaccaa gacctcggac accgtgtgtg actcctgtga ggacagcaca     180

tacacccagc tctggaactg ggttcccgag tgcttgagct gtggctcccg ctgtagctct     240

gaccaggtgg aaactcaagc ctgcactcgg gaacagaacc gcatctgcac ctgcaggccc     300

ggctggtact gcgcgctgag caagcaggag gggtgccggc tgtgcgcgcc gctgcgcaag     360

tgccgcccgg gcttcggcgt ggccagacca ggaactgaaa catcagacgt ggtgtgcaag     420

ccctgtgccc cggggacgtt ctccaacacg acttcatcca cggatatttg caggccccac     480

cagatctgtt aa                                                         492


<210>  85
<211>  31
<212>  DNA
<213>  Artificial

<220>
<223>  Oligonucleotide primer

<400>  85
cccaccagat ctgttgcgac aagacccaca c                                     31


<210>  86
<211>  31
<212>  DNA
<213>  Artificial

<220>
<223>  Oligonucleotide primer

<400>  86
gtgtgggtct tgtcgcaaca gatctggtgg g                                     31


<210>  87
<211>  1194
<212>  DNA
<213>  Artificial

<220>
<223>  Pro_TNFaR1b P24_C185_hinge_SHBG construct

<400>  87
atgcccgccc aggtggcatt tacaccctac gccccggagc ccgggagcac atgccggctc      60

agagaatact atgaccagac agctcagatg tgctgcagca aatgctcgcc gggccaacat     120

gcaaaagtct tctgtaccaa gacctcggac accgtgtgtg actcctgtga ggacagcaca     180

tacacccagc tctggaactg ggttcccgag tgcttgagct gtggctcccg ctgtagctct     240

gaccaggtgg aaactcaagc ctgcactcgg gaacagaacc gcatctgcac ctgcaggccc     300

ggctggtact gcgcgctgag caagcaggag gggtgccggc tgtgcgcgcc gctgcgcaag     360

tgccgcccgg gcttcggcgt ggccagacca ggaactgaaa catcagacgt ggtgtgcaag     420

ccctgtgccc cggggacgtt ctccaacacg acttcatcca cggatatttg caggccccac     480

cagatctgtt gcgacaagac ccacacctgc cccccctgcc ccgcccccga gctgctgggc     540

ggcccctccg tgttcctgtt cccccccaag cccaaggata ccaaccctaa ggatgactgg     600

tttatgctgg gacttcgaga cggcaggcct gagatccaac tgcacaatca ctgggcccag     660

cttacggtgg gtgctggacc acggctggat gatgggagat ggcaccaggt ggaagtcaag     720

atggaggggg actctgtgct gctggaggtg gatggggagg aggtgctgcg cctgagacag     780

gtctctgggc ccctgaccag caaacgccat cccatcatga ggattgcgct tggggggctg     840

ctcttccccg cttccaacct tcggttgccg ctggttcctg ccctggatgg ctgcctgcgc     900

cgggattcct ggctggacaa acaggccgag atctcagcat ctgcccccac tagcctcaga     960

agctgtgatg tagaatcaaa tcccgggata tttctccctc cagggactca ggcagaattc    1020

aatctccgag acattcccca gcctcatgca gagccctggg ccttctcttt ggacctggga    1080

ctcaagcagg cagcaggctc aggccacctc cttgctcttg ggacaccaga gaacccatct    1140

tggctcagtc tccacctcca agatcaaaag gtggtgttgt cttctgggtc gtga          1194

<210>  88
<211>  397
<212>  PRT
<213>  Artificial

<220>
<223>  Pro_TNFaR1b P24_C185_hinge_SHBG protein

<400>  88

Met Pro Ala Gln Val Ala Phe Thr Pro Tyr Ala Pro Glu Pro Gly Ser
1               5                   10                  15

Thr Cys Arg Leu Arg Glu Tyr Tyr Asp Gln Thr Ala Gln Met Cys Cys
            20                  25                  30

Ser Lys Cys Ser Pro Gly Gln His Ala Lys Val Phe Cys Thr Lys Thr
        35                  40                  45

Ser Asp Thr Val Cys Asp Ser Cys Glu Asp Ser Thr Tyr Thr Gln Leu
    50                  55                  60

Trp Asn Trp Val Pro Glu Cys Leu Ser Cys Gly Ser Arg Cys Ser Ser
65                  70                  75                  80

Asp Gln Val Glu Thr Gln Ala Cys Thr Arg Glu Gln Asn Arg Ile Cys
                85                  90                  95

Thr Cys Arg Pro Gly Trp Tyr Cys Ala Leu Ser Lys Gln Glu Gly Cys
            100                 105                 110

Arg Leu Cys Ala Pro Leu Arg Lys Cys Arg Pro Gly Phe Gly Val Ala
        115                 120                 125

Arg Pro Gly Thr Glu Thr Ser Asp Val Val Cys Lys Pro Cys Ala Pro
    130                 135                 140

Gly Thr Phe Ser Asn Thr Thr Ser Ser Thr Asp Ile Cys Arg Pro His
145                 150                 155                 160

Gln Ile Cys Cys Asp Lys Thr His Thr Cys Pro Pro Cys Pro Ala Pro
                165                 170                 175

Glu Leu Leu Gly Gly Pro Ser Val Phe Leu Phe Pro Pro Lys Pro Lys
            180                 185                 190

Asp Thr Asn Pro Lys Asp Asp Trp Phe Met Leu Gly Leu Arg Asp Gly
        195                 200                 205

Arg Pro Glu Ile Gln Leu His Asn His Trp Ala Gln Leu Thr Val Gly
    210                 215                 220

Ala Gly Pro Arg Leu Asp Asp Gly Arg Trp His Gln Val Glu Val Lys
225                 230                 235                 240

Met Glu Gly Asp Ser Val Leu Leu Glu Val Asp Gly Glu Glu Val Leu
                245                 250                 255

Arg Leu Arg Gln Val Ser Gly Pro Leu Thr Ser Lys Arg His Pro Ile
            260                 265                 270

Met Arg Ile Ala Leu Gly Gly Leu Leu Phe Pro Ala Ser Asn Leu Arg
        275                 280                 285

Leu Pro Leu Val Pro Ala Leu Asp Gly Cys Leu Arg Arg Asp Ser Trp
    290                 295                 300

Leu Asp Lys Gln Ala Glu Ile Ser Ala Ser Ala Pro Thr Ser Leu Arg
305                 310                 315                 320

Ser Cys Asp Val Glu Ser Asn Pro Gly Ile Phe Leu Pro Pro Gly Thr
                325                 330                 335

Gln Ala Glu Phe Asn Leu Arg Asp Ile Pro Gln Pro His Ala Glu Pro
            340                 345                 350

Trp Ala Phe Ser Leu Asp Leu Gly Leu Lys Gln Ala Ala Gly Ser Gly
        355                 360                 365

His Leu Leu Ala Leu Gly Thr Pro Glu Asn Pro Ser Trp Leu Ser Leu
    370                 375                 380

His Leu Gln Asp Gln Lys Val Val Leu Ser Ser Gly Ser
385                 390                 395

<210>  89
<211>  877
<212>  DNA
<213>  Artificial

<220>
<223>  Pro_TNFaR1b S71-C126_hinge_SHBG construct

<400>  89
atgtcggaca ccgtgtgtga ctcctgtgag gacagcacat acacccagct ctggaactgg      60

gttcccgagt gcttgagctg tggctcccgc tgtagctctg accaggtgga aactcaagcc     120

tgcactcggg aacagaaccg catctgcacc tgcaggcccg gctggtactg ctgcgacaag     180

acccacacct gccccccctg ccccgccccc gagctgctgg gcggcccctc cgtgttcctg     240

ttccccccca agcccaagga taccaaccct aaggatgact ggtttatgct gggacttcga     300

gacggcaggc ctgagatcca actgcacaat cactgggccc agcttacggt gggtgctgga    360

ccacggctgg atgatgggag atggcaccag gtggaagtca agatggaggg ggactctgtg    420

ctgctggagg tggatgggga ggaggtgctg cgcctgagac aggtctctgg gcccctgacc    480

agcaaacgcc atcccatcat gaggattgcg cttggggggc tgctcttccc cgcttccaac    540

cttcggttgc cgctggttcc tgccctggat ggctgcctgc gccgggattc ctggctggac    600

aaacaggccg agatctcagc atctgccccc actagcctca gaagctgtga tgtagaatca    660

aatcccggga tatttctccc tccagggact caggcagaat tcaatctccg agacattccc    720

cagcctcatg cagagccctg ggccttctct ttggacctgg gactcaagca ggcagcaggc    780

tcaggccacc tccttgctct tgggacacca gagaacccat cttggctcag tctccacctc    840

caagatcaaa aggtggtgtt gtcttctggg tcgtgaa                             877

<210>  90
<211>  291
<212>  PRT
<213>  Artificial

<220>
<223>  Pro_TNFaR1b S71-C126_hinge_SHBG protein

<400>  90

Met Ser Asp Thr Val Cys Asp Ser Cys Glu Asp Ser Thr Tyr Thr Gln
1               5                   10                  15

Leu Trp Asn Trp Val Pro Glu Cys Leu Ser Cys Gly Ser Arg Cys Ser
            20                  25                  30

Ser Asp Gln Val Glu Thr Gln Ala Cys Thr Arg Glu Gln Asn Arg Ile
        35                  40                  45

Cys Thr Cys Arg Pro Gly Trp Tyr Cys Cys Asp Lys Thr His Thr Cys
    50                  55                  60

Pro Pro Cys Pro Ala Pro Glu Leu Leu Gly Gly Pro Ser Val Phe Leu
65                  70                  75                  80

Phe Pro Pro Lys Pro Lys Asp Thr Asn Pro Lys Asp Asp Trp Phe Met
                85                  90                  95

Leu Gly Leu Arg Asp Gly Arg Pro Glu Ile Gln Leu His Asn His Trp

            100                 105                 110

Ala Gln Leu Thr Val Gly Ala Gly Pro Arg Leu Asp Asp Gly Arg Trp
        115                 120                 125

His Gln Val Glu Val Lys Met Glu Gly Asp Ser Val Leu Leu Glu Val
    130                 135                 140

Asp Gly Glu Glu Val Leu Arg Leu Arg Gln Val Ser Gly Pro Leu Thr
145                 150                 155                 160

Ser Lys Arg His Pro Ile Met Arg Ile Ala Leu Gly Gly Leu Leu Phe
                165                 170                 175

Pro Ala Ser Asn Leu Arg Leu Pro Leu Val Pro Ala Leu Asp Gly Cys
            180                 185                 190

Leu Arg Arg Asp Ser Trp Leu Asp Lys Gln Ala Glu Ile Ser Ala Ser
        195                 200                 205

Ala Pro Thr Ser Leu Arg Ser Cys Asp Val Glu Ser Asn Pro Gly Ile
    210                 215                 220

Phe Leu Pro Pro Gly Thr Gln Ala Glu Phe Asn Leu Arg Asp Ile Pro
225                 230                 235                 240

Gln Pro His Ala Glu Pro Trp Ala Phe Ser Leu Asp Leu Gly Leu Lys
                245                 250                 255

Gln Ala Ala Gly Ser Gly His Leu Leu Ala Leu Gly Thr Pro Glu Asn
            260                 265                 270

Pro Ser Trp Leu Ser Leu His Leu Gln Asp Gln Lys Val Val Leu Ser
        275                 280                 285

Ser Gly Ser
    290

<210> 91
<211> 939
<212> DNA
<213> Artificial

<220>
<223>  Euk_TNFaR1b S71-C126_hinge_SHBG construct

<400>  91
atggcgcccg tcgccgtctg ggccgcgctg gccgtcggac tggagctctg ggctgcggcg      60

cacgcctcgg acaccgtgtg tgactcctgt gaggacagca catacaccca gctctggaac     120

tgggttcccg agtgcttgag ctgtggctcc cgctgtagct ctgaccaggt ggaaactcaa     180

gcctgcactc gggaacagaa ccgcatctgc acctgcaggc ccggctggta ctgctgcgac     240

aagacccaca cctgcccccc ctgccccgcc cccgagctgc tgggcggccc ctccgtgttc     300

ctgttccccc ccaagcccaa ggataccaac cctaaggatg actggtttat gctgggactt     360

cgagacggca ggcctgagat ccaactgcac aatcactggg cccagcttac ggtgggtgct     420

ggaccacggc tggatgatgg gagatggcac caggtggaag tcaagatgga gggggactct     480

gtgctgctgg aggtggatgg ggaggaggtg ctgcgcctga gacaggtctc tgggcccctg     540

accagcaaac gccatcccat catgaggatt gcgcttgggg ggctgctctt ccccgcttcc     600

aaccttcggt tgccgctggt tcctgccctg gatggctgcc tgcgccggga ttcctggctg     660

gacaaacagg ccgagatctc agcatctgcc cccactagcc tcagaagctg tgatgtagaa     720

tcaaatcccg ggatatttct ccctccaggg actcaggcag aattcaatct ccgagacatt     780

ccccagcctc atgcagagcc ctgggccttc tctttggacc tgggactcaa gcaggcagca     840

ggctcaggcc acctccttgc tcttgggaca ccagagaacc catcttggct cagtctccac     900

ctccaagatc aaaaggtggt gttgtcttct gggtcgtga                            939


<210>  92
<211>  313
<212>  PRT
<213>  Artificial

<220>
<223>  Euk_TNFaR1b S71-C126_hinge_SHBG protein

<400>  92

Thr Met Ala Pro Val Ala Val Trp Ala Ala Leu Ala Val Gly Leu Glu
1               5                   10                  15


Leu Trp Ala Ala Ala His Ala Ser Asp Thr Val Cys Asp Ser Cys Glu
            20                  25                  30


Asp Ser Thr Tyr Thr Gln Leu Trp Asn Trp Val Pro Glu Cys Leu Ser

35                  40                  45

Cys Gly Ser Arg Cys Ser Ser Asp Gln Val Glu Thr Gln Ala Cys Thr
    50                  55                  60

Arg Glu Gln Asn Arg Ile Cys Thr Cys Arg Pro Gly Trp Tyr Cys Cys
65                  70                  75                  80

Asp Lys Thr His Thr Cys Pro Pro Cys Pro Ala Pro Glu Leu Leu Gly
                85                  90                  95

Gly Pro Ser Val Phe Leu Phe Pro Pro Lys Pro Lys Asp Thr Asn Pro
            100                 105                 110

Lys Asp Asp Trp Phe Met Leu Gly Leu Arg Asp Gly Arg Pro Glu Ile
        115                 120                 125

Gln Leu His Asn His Trp Ala Gln Leu Thr Val Gly Ala Gly Pro Arg
    130                 135                 140

Leu Asp Asp Gly Arg Trp His Gln Val Glu Val Lys Met Glu Gly Asp
145                 150                 155                 160

Ser Val Leu Leu Glu Val Asp Gly Glu Glu Val Leu Arg Leu Arg Gln
                165                 170                 175

Val Ser Gly Pro Leu Thr Ser Lys Arg His Pro Ile Met Arg Ile Ala
            180                 185                 190

Leu Gly Gly Leu Leu Phe Pro Ala Ser Asn Leu Arg Leu Pro Leu Val
        195                 200                 205

Pro Ala Leu Asp Gly Cys Leu Arg Arg Asp Ser Trp Leu Asp Lys Gln
    210                 215                 220

Ala Glu Ile Ser Ala Ser Ala Pro Thr Ser Leu Arg Ser Cys Asp Val
225                 230                 235                 240

Glu Ser Asn Pro Gly Ile Phe Leu Pro Pro Gly Thr Gln Ala Glu Phe
                245                 250                 255

Asn Leu Arg Asp Ile Pro Gln Pro His Ala Glu Pro Trp Ala Phe Ser

260                 265                 270

Leu Asp Leu Gly Leu Lys Gln Ala Ala Gly Ser Gly His Leu Leu Ala
        275                 280                 285

Leu Gly Thr Pro Glu Asn Pro Ser Trp Leu Ser Leu His Leu Gln Asp
    290                 295                 300

Gln Lys Val Val Leu Ser Ser Gly Ser
305                 310

<210>  93
<211>  444
<212>  DNA
<213>  Homo sapiens

<400>  93
atgggcctct ccaccgtgcc tgacctgctg ctgccactgg tgctcctgga gctgttggtg      60

ggaatatacc cctcaggggt tattggactg gtccctcacc taggggacag ggagaagaga     120

gatagtgtgt gtccccaagg aaaatatatc caccctcaaa ataattcgat ttgctgtacc     180

aagtgccaca aaggaaccta cttgtacaat gactgtccag gcccggggca ggatacggac     240

tgcagggagt gtgagagcgg ctccttcacc gcttcagaaa accacctcag acactgcctc     300

agctgctcca aatgccgaaa ggaaatgggt caggtggaga tctcttcttg cacagtggac     360

cgggacaccg tgtgtggctg caggaagaac cagtaccggc attattggag tgaaaacctt     420

ttccagtgct tcaattgcag cctc                                            444

<210>  94
<211>  501
<212>  DNA
<213>  Homo sapiens

<400>  94
atgggcctct ccaccgtgcc tgacctgctg ctgccactgg tgctcctgga gctgttggtg      60

ggaatatacc cctcaggggt tattggactg gtccctcacc taggggacag ggagaagaga     120

gatagtgtgt gtccccaagg aaaatatatc caccctcaaa ataattcgat ttgctgtacc     180

aagtgccaca aaggaaccta cttgtacaat gactgtccag gcccggggca ggatacggac     240

tgcagggagt gtgagagcgg ctccttcacc gcttcagaaa accacctcag acactgcctc     300

agctgctcca aatgccgaaa ggaaatgggt caggtggaga tctcttcttg cacagtggac     360

cgggacaccg tgtgtggctg caggaagaac cagtaccggc attattggag tgaaaacctt 420

ttccagtgct tcaattgcag cctctgcctc aatgggaccg tgcacctctc ctgccaggag 480

aaacagaaca ccgtgtgcac c 501

<210> 95
<211> 603
<212> DNA
<213> Homo sapiens

<400> 95
atgggcctct ccaccgtgcc tgacctgctg ctgccactgg tgctcctgga gctgttggtg 60

ggaatatacc cctcaggggt tattggactg gtccctcacc taggggacag ggagaagaga 120

gatagtgtgt gtccccaagg aaaatatatc caccctcaaa ataattcgat ttgctgtacc 180

aagtgccaca aaggaaccta cttgtacaat gactgtccag gcccggggca ggatacggac 240

tgcagggagt gtgagagcgg ctccttcacc gcttcagaaa accacctcag acactgcctc 300

agctgctcca aatgccgaaa ggaaatgggt caggtggaga tctcttcttg cacagtggac 360

cgggacaccg tgtgtggctg caggaagaac cagtaccggc attattggag tgaaaacctt 420

ttccagtgct tcaattgcag cctctgcctc aatgggaccg tgcacctctc ctgccaggag 480

aaacagaaca ccgtgtgcac ctgccatgca ggtttctttc taagagaaaa cgagtgtgtc 540

tcctgtagta actgtaagaa aagcctggag tgcacgaagt tgtgcctacc ccagattgag 600

aat 603

## Description

The present invention relates to fusion polypeptides comprising a biologically active component and a dimerization component, wherein said dimerization component is derived from sex hormone-binding globulin (SHBG). The invention further relates to nucleic acid molecules encoding said fusion polypeptides as well as methods of producing the fusion polypeptides. The fusion polypeptides of this invention are useful in the field of diagnostics and therapy.

### BACKGROUND OF THE INVENTION

Biologically active proteins or polypeptides can be useful for diagnosis and therapy of a large number of diseases and disorders. The efficiency of such biologically active proteins depends on parameters like stability and half life of the biological molecule in biological fluids such as serum.

One example of such biologically active proteins are antibodies which can be used to target tumors in order to deliver specific agents such as cytotoxins or enzymes to the tumor for treating a disease caused by the tumor. Recombinant antibodies have been used for this purpose but due to their fast clearance from blood circulation such approaches result in low total dose accumulation (see e.g. BEGENT et al. (1996), Nat. Med. 2, 979-984).

This problem can partly be solved by fusing the antibody or another biologically active polypeptide to a carrier polypeptide. Approaches using human serum albumin or a fragment of human serum albumin as a carrier protein demonstrated that the resulting biologically active fusion protein has a prolonged shelf life, an increased plasma stability and is more stable in solution (see e.g. US 2007/0048282).

Another approach uses recombinant antibody molecules having the biologically active substance substituted for the variable domains of the immunoglobulin molecule heavy and light chains (see WO 94/06476). This approach provides a dimeric platform structure, but has the disadvantage of very complex recombinant fusion proteins, which are typically characterised by low yields in most expression systems.

A third approach uses the homotrimeric protein tetranectin, (see WO 2006/053568). Tetranectin is physiologically present in blood plasma, but its physiological role is still not fully clear, so that the consequences of using tetranectin as a carrier for long term applications are not known.

Another approach uses a peptide capable of dimerisation carrying a Fv-fragment of a recombinant antibody on at least one of the two monomeric fusion proteins (see EP 0 654 085).

Therefore, several problems can lead to dysfunction of the fusion constructs.

Typically, the antigenicity of the construct is critical since the non-physiological combination of the biologically active polypeptide and the carrier molecule can lead to increased antigenicity of the construct. Furthermore, many of the biologically active fusion polypeptides have a relatively high molecular weight which may lead to low yield when the biologically active fusion protein is produced by the cellular expression system.

In all cellular systems (from prokaryotes to eukaryotes), proteins that are destined for export into the extracellular space often exhibit multiple disulfide bonds with complex folding patterns. Having multiple disulfide bridges stabilizes the secondary and tertiary structures of these secreted proteins, conferring a survival advantage in the harsher microenvironment outside the intracellular compartment.

Native disulphide bond formation is a complex process. Disulphide bonds must not only be formed (oxidation), but also incorrect bonds must be broken (reduction) or rearranged (isomerization) (Lars Ellgaard & Lloyd W.Ruddock EMBO reports (2005) 6, 28-32).

Expression of proteins with disufide bonds in prokaryotic systems leads to insoluble recombinant protein in inclusion bodies, whereas the eukaryotic cells are strongly burdened with the production of these proteins (for a review, see Tu & Weissman, 2004, J Cell Biol 164(3):341-6).

Multimerising peptides are also used as a carrier for miniantibodies. Use of a multimeric platform can be advantageous in two ways. Firstly, biologically active components consisting of two or more subunits can be applied in a multimeric form. Secondly, combinations of biologically active components can be applied.

Polymerising proteins show a significantly greater availability for ligand binding. This has been shown for antibodies which consist of two monomers. It has been shown that complete antibody molecules which are dimers (two monomers in complex) have 10-30-fold increases in avidity over monomer antibody fragments.

Thus, there is a need for biologically active fusion polypeptides which do not exhibit the above drawbacks and which provide stable biologically active fusion polypeptides.

This problem is solved by the nucleic acid molecules of the present invention and the fusion polypeptides encoded by them.

According to one aspect of the present invention a nucleic acid molecule is provided which encodes a fusion polypeptide, which fusion polypeptide comprises the following fusion polypeptide components:
(a) a biologically active component; and
(b) a dimerization component,
which dimerization component (b) is capable of forming dimers with a dimerization component (b) comprised in another of said fusion polypeptides, wherein said dimerization component is derived from sex hormone-binding globulin (SHBG).

The sex hormone-binding globulin (SHGB) transports testosterone and estradiol in the blood. Human SHBG is a homodimeric plasma glycoprotein produced by hepatocytes under the control of various hormonal and metabolic regulators. SHBG contains the coding sequence for the secretion signal polypeptide and two contiguous laminin G-like (LG) domains. The amino-terminal LG domain contains the steroid-binding site, the dimer interface, and several cation-binding sites (Grishkovskaya et al, 2000, EMBO J. 2000 February 15; 19(4): 504-512; Avvakumov et al, 2002 J Biol Chem. 2002 Nov 22;277(47):45219-25).

The carboxy-terminal LG domain of SHBG normally contains two sites for N-glycosylation, one of which is invariably conserved in SHBG molecules across a wide range of mammalian species. A variant human SHBG, with a point mutation in exon 8 encoding an amino acid substitution (Asp327Asn), which introduces an additional consensus site for N-glycosylation, shows a significantly higher biological half life than heterozygous or wild type human SHBG (Cousin et al., 1998, J Clin Endocrinol Metab. 1998 Jan;83(1):235-40). A site for O-glycosylation is present at the amino terminus.

The term "derived from" relates to polypeptides which may differ from the original polypeptide by the addition, deletion, substitution or insertion of amino acids, or by the linkage of other molecules to the encoded polypetide without losing the function of the original polypeptide.

A dimerization component which is derived from SHBG is advantageous compared to other dimerization components used in the art since it has been surprisingly found that dimerization domains of SHBG interact spontaneously and very strongly. Furthermore, these domains have a relatively low molecular weight.

A "dimerization component derived from SHBG" as used herein means each polypeptide fragment of SHBG which is able to form stable dimers as well as polypeptide fragments which have a sequence similarity of at least 50%, preferably at least 60%, preferred at least 70%, more preferred at least 80%, even more preferred at least 90% and most preferred at least 95% to the SHBG dimerization domain shown in SEQ ID NO:2 and which have the ability to form stable dimers.

The "sequence similarity" can be determined by standard bioinformatic programs known by the person skilled in the art, e.g the BLAST, FASTA or ClustalW programs.

In a preferred embodiment of the invention the nucleic acid molecule encodes a dimerization component derived from SHBG wherein the nucleic acid encoding said dimerization component comprises the nucleic acid sequence as shown in SEQ ID NO:1 and/or comprises a fragment of at least 50 nucleotides of SEQ ID NO:1 and/or has a nucleic acid sequence similarity of at least 70%, preferably 80%, more preferred 90% and most preferred 95% to SEQ ID NO:1.

In a further preferred embodiment the nucleic acid molecule encodes a dimerization component derived from SHBG which is capable of forming dimers with a dissociation constant K_{D} of at least 10⁻¹⁰, preferably at least 10⁻¹¹ and most preferred 10-¹².

The term "dissociation constant" as used herein describes the affinity between two dimerization components. The K_{D} value is influenced by non-covalent intermolecular interactions between the two molecules such as hydrogen bonding, electrostatic interactions, hydrophobic and Van der Waals forces and specifies how tightly these two molecules bind each other.

In general, the smaller the dissociation constant (represented in molar units (M)), the more tightly bound the molecules are, or the higher the affinity between the two molecules.

A "stable dimer" in the context of the present invention is a dimer having a dissociation constant K_{D} of at least 10⁻⁸ as determined under standard conditions.

In another preferred embodiment the nucleic acid molecule encodes a dimerization component derived from SHBG wherein the nucleic acid encoding said dimerization component consists of the nucleic acid sequence as shown in SEQ ID NO:1.

In another preferred embodiment the present invention is directed to the nucleic acid molecule of the invention wherein the dimerization component encoded by said nucleic acid is a chimeric construct comprising at least two parts of the dimerization domain of SHBG as shown in SEQ ID NO:1 which are naturally not adjacent to each other. In a preferred embodiment the dimerization component is a chimeric construct consisting of at least two parts of the dimerization domain of SHBG which are naturally not adjacent to each other.

"Adjacent" as used herein means that the two parts are normally in close proximity to each other. This can be due to primary structure of a protein, i.e. the two parts are consecutive in the amino acid sequence and it can also be the result of the folding of the mature protein. In the folded protein, also referred to as tertiary structure, two parts of the protein may come into close proximity which are considerably separated on the primary structure level.

A further preferred embodiment relates to the nucleic acid molecule of the invention wherein the dimerization component derived from sex hormone-binding globulin (SHBG) is a chimeric construct consisting of at least two parts, one of which is derived from the dimerization domain of SHBG, whereas the at least one further part is derived from a domain of SHBG other than the dimerization domain, wherein the at least two parts are naturally not adjacent to each other.

Another preferred embodiment relates to the nucleic acid molecule of the invention wherein the dimerization component derived from sex hormone-binding globulin (SHBG) is a chimeric construct consisting of at least two parts, one of which is derived from the dimerization domain of SHBG, whereas the at least one further part is derived from another protein. According to a further embodiment the at least one further part is derived from the group of steroid hormone binding proteins. In a preferred embodiment the at least one further part is derived from cortisol-binding globulin (CBG).

The nucleic acid molecules of the present invention further encode a biologically active component.

A "biologically active component" according to this invention refers to proteins, polypeptides, antibodies, antibody fragments, for example Fab, Fab', F(ab')₂, and Fv fragments, diabodies, linear antibodies, single-chain antibodies, domain antibodies, nanobodies molecules such as e.g. scFv and multispecific antibodies formed from antibody fragments. The biologically active components possess at least one biological activity, e.g. the ability to bind specifically to a particular cell type and/or to function in signal transduction pathways.

In a preferred embodiment of the invention the biologically active component is a ligand binding component, a subtrate-binding component or a substrate-component.

According to the present invention "ligand binding" components are proteins or fragments thereof that specifically bind to target ligand molecules. Ligand binding components comprise receptors, carrier proteins, hormones, cellular adhesive proteins, i.e. proteins that direct or induce the adhesion of one cell to another, lectin binding molecules, growth factors and the like.

According to the present invention "subtrate binding components" are proteins or fragments thereof that specifically bind to a substrate, e.g. a substrate of a specific enzyme. Therefore, enzymes are preferred substrate binding components of the present invention.

According to the present invention "substrate components" are molecules that are acted upon by an enzyme. Preferred substrate components according to the invention are nucleotides, polynucleotides, peptides, proteins, carbohydrates or lipids.

In another preferred embodiment the ligand binding component is derived from a cytokine, a growth factor, a regulatory peptide factor, a hormone, a receptor or a fragment of these.

In an especially preferred embodiment the ligand binding component and/or subtrate binding component and/or subtrate component is selected or derived from the group consisting of IL-1, IL-1R, IL-2, IL-2R, IL-3, IL-3R, IL-4, IL-4R, IL-5, IL-5R, IL-6, IL-6R, IL-7, IL-7R, IL-8, IL-9, IL-9R, IL-10, IL-11, IL-12, IL-13, IL-13R, IL-14, IL-15, IL-15R, IL-16, IL-17, IL-35, TNF, TGF, TGF-alpha, IFN, GM-CSF, GM-CSFR, G-CSF, G-CSFR, EPO, EPOR, TPO, M-CSF, GHR, TNFR, TGFR, IFNR, interferon-α R, interferon-**β** R, interferon-γ R, cMp1, gp130, Fas (Apo 1), CCR1, CXCR1-4, TrkA, TrkB, TrkC, Htk, REK7, Rse/Tyro-3, hepatocyte growth factor R, platelet-derived growth factor R, Flt-1, CD2, CD4, CD5, CD6, CD22, CD27, CD28, CD30, CD31, CD40, CD44, CD100, CD137, CD150, LAG-3, B7, B61, **β**-neurexin, CTLA-4, ICOS, ICAM-1, complement R-2 (CD21) IgER, lysosomal membrane gp1, α2 microglobulin receptor related proteins and sodium-releasing peptide R, LIF, LT, FGF, VEGF, EGF, SCF, oncostatin M, Amphiregulin, Mullerian-inhibiting substance, BCGF, MIF, Endostatin and Angiostatin, Adiponectin, GLP, GLP2, PACAP, VIP, CD4 cluster of differentiation, Secretin, glicentin, Oxyntomodulin, ANP, BNP, Interferone, BDNF, NGF, GDNF, Somatostatin, Apolipoprotein A-I, ApoA-I Milano, Endostatin, Angiostatin, enzymes from the group of hydrolases (including phosphatases, nucleases phosphodiesterases), oxidoreductases, transferases (including phosphotransferases), lyases, isomerases and ligases and substrates of these enzymes including nucleotides, proteins, carbohydrates and lipids or biologically active fragments, variants, mutants, analogs or derivatives thereof.

In the context of this invention, "active" fragments, variants, mutants, analogs or derivatives are defined as molecules that retain the specific ligand binding function to particular ligands of the molecule they are derived from.

The term "active fragment" means a fragment of a protein that has full or partial biological activity of the entire protein.

The term "variant" means any polypeptide having one or several structural modification(s) introduced therein, such as mutation(s), deletion(s), substitution(s) and/or addition(s) of one or several amino acid residues, the variant still exhibiting the biological activity.

With respect to proteins the term "mutant" means a modified version of the native protein. A protein may be modified by amino acid substitution, deletion, addition, permutation (e.g., circular permutation), etc. An "active" mutant retains a biological characteristic of the native protein (e.g., the capability of binding of a ligand).

The term "analog" means any structurally or functionally related compound which mimicks the biological activity of the ligand binding components mentioned above.

In an especially preferred embodiment the nucleic acid molecule of the invention encodes a ligand binding component which comprises the p75 fragment of the TNF receptor encoded by the nucleic acid sequence as shown in SEQ ID NO:3 or a sequence having a sequence similarity of at least 60%, preferably 70%, more preferred 80%, even more preferred 90% and most preferred 95% to SEQ ID NO:3. SEQ ID NO:4 shows the corresponding amino acid sequence of the p75 fragment of TNF receptor encoded by the nucleic acid of SEQ ID NO:3.

Another embodiment of the invention is directed to a nucleic acid molecule encoding a fusion polypeptide, which fusion polypeptide comprises the following fusion polypeptide components:
(a) a ligand binding component; and
(b) a dimerization component,
wherein the nucleic acid encoding the ligand binding component comprises the fragment of human TNF receptor p75 as shown in SEQ ID NO:3 or a sequence having a sequence similarity of at least 70%, more preferred 80% and most preferred 95% to SEQ ID NO:3 and wherein the dimerization component can be any component which is capable to form stable dimers.

In a preferred embodiment the nucleic acid encoding the ligand binding component comprises the fragment of human TNF receptor p75 selected from the list consisting of SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, and SEQ ID NO:84.

In an especially preferred embodiment said dimerization component is selected from the group consisting of SHBG dimerization domain, leucine zipper, an immunglobuline derived domain, e.g. the Fc domain of IgG, the heavy chain of IgG and the light chain of IgG. In a more preferred embodiment said dimerization component comprises the SHBG dimerization domain encoded by the nucleic acid sequence as shown in SEQ ID NO:1.

Another embodiment of the invention is directed to a nucleic acid molecule encoding a fusion polypeptide, which fusion polypeptide comprises the following fusion polypeptide components:
(a) a ligand binding component; and
(b) a dimerization component,
wherein the nucleic acid encoding the ligand binding component comprises a fragment of human TNF receptor p55 selected from the list consisting of SEQ ID NO:31, SEQ ID NO:32 and SEQ ID NO:33, SEQ ID NO:93, SEQ ID NO:94 and SEQ ID NO:95, and wherein the dimerization component can be any component which is capable to form stable dimers.

In a preferred embodiment said dimerization component is selected from the group consisting of SHBG dimerization domain, leucine zipper, an immunglobuline derived domain, e.g. the Fc domain of IgG, the heavy chain of IgG and the light chain of IgG. In an especially preferred embodiment said dimerization component comprises the SHBG dimerization domain encoded by the nucleic acid sequence as shown in SEQ ID NO:1.

In another embodiment of the invention the nucleic acid molecules encoding the fusion polypeptides further encode a linker peptide which is located between components (a) and (b) of the fusion polypeptides.

Suitable linker peptides will adopt a flexible extended conformation. Therefore, the linker peptide is preferably a flexible linker. The flexible molecular linkers are preferably inert, hydrophilic and non-cleavable by proteases. The flexible molecular linkers are also preferably designed to lack secondary structure under physiological conditions. Thus, for example, the polypeptide linker sequences are preferably composed of a plurality of residues selected from the group consisting of glycine, serine, threonine, cysteine, asparagine, glutamine and proline. Particularly preferred are polypeptide linkers consisting essentially of cysteine, alanine and proline residues.

In a preferred embodiment of the invention the linker peptide is flexible having a length of at least 10 amino acids up to 125 amino acids and is selected from the group consisting of Fc hinge, Ig hinge, endosomal escape domain, Glycin-linker, or fragments thereof.

In another preferred embodiment of the invention the linker peptide is a linker consisting of four glycine residues and one serine residue (Gly Gly Gly Gly Ser) and concatameres thereof.

In an especially preferred embodiment the linker peptide is an IG hinge encoded by a nucleic acid sequence as shown in SEQ ID NO:21. The amino acid sequence of the linker peptide encoded by SEQ ID NO:21 is shown in SEQ ID NO:22.

In a preferred embodiment the nucleic acids of the present invention are provided in isolated form.

The invention is further directed to a vector comprising the nucleic acid molecules of the invention. In a preferred embodiment the vector is an expression vector comprising a nucleic acid molecule of the invention, wherein the nucleic acid molecule is operatively linked to an expression control sequence. Expression control sequences which are suitable for the present invention are for example RSV control sequences, CMV control sequences, retroviral LTR sequences, SV-40 control sequences, p-actin control sequences, T7 control sequences and lac (bla) control sequences, mouse WAP promoter, cauliflower mosaic virus(CaMV) 35S promoter, nopaline synthase (nos) promoter, the Figwort mosaic virus 35S promoter, the sugarcane bacilliform virus promoter, the commelina yellow mottle virus promoter, the light-inducible promoter from the small subunit of the ribulose-1,5-bis-phosphate carboxylase (ssRUBISCO), the rice cytosolic triosephosphate isomerase (TPI) promoter, the adenine phosphoribosyltransferase (APRT) promoter of Arabidopsis, the rice actin 1 gene promoter, and the mannopine synthase, octopine synthase promoters.

The invention is further directed to a host cell, a host animal or a host plant, respectively, for production of the fusion polypeptides of the invention, wherein said host cell, host animal or host plant comprises the expression vector of the invention which has been introduced into the host cell, the host animal or the host plant.

In a preferred embodiment of the invention the host cell is a bacterial cell, a yeast cell, an insect cell or a mammalian cell. An especially preferred host cell is an *E.coli* cell, a Chinese Hamster ovary (CHO) cell, a 293 cell or a K562 cell.

In a preferred embodiment of the invention the host animal is a mammalian animal. An especially preferred host mammalian animal is a milk producing animal.

The present invention is also directed to a method of producing the fusion polypeptides of the invention. The method comprises growing the host cell of the invention comprising an expression vector of the invention, wherein said growing is carried out under conditions permitting production of the fusion polypeptides and wherein the fusion polypeptides, produced by the host cell, are recovered from the host cell and/or from the medium used for growing the host cell.

The present invention is also directed to another method of producing the fusion polypeptides of the invention. The method comprises expressing the fusion polypeptides in a cell free expression system using an expression vector comprising a nucleic acid molecule of the invention, wherein the nucleic acid molecule is operatively linked to an expression control sequence.

The invention is also directed to the fusion polypeptides encoded by the nucleic acid molecules of the invention. The fusion polypeptides can be in monomeric form. In a preferred embodiment of the invention the fusion polypeptides are present in dimeric form. The invention also comprises fusion polypeptides in multimeric form.

Another preferred embodiment of the invention is directed to a fusion polypeptide in monomeric or dimeric form obtained by the methods of the invention.

The monomers of a dimeric fusion polypeptide may comprise the same biologically active component or different biologically active components.

The invention is also directed to fusion polypeptides in dimeric form, wherein only one of the two monomers of the fusion polypeptide in dimeric form comprises a biologically active component according to the invention.

A preferred fusion polypeptide comprises the p75 fragment of human TNF receptor as shown in SEQ ID NO:4 and the dimerization domain of SHBG. In an especially preferred embodiment the fusion polypeptide consists of the p75 fragment of TNF receptor as shown in SEQ ID NO:4 and the dimerization domain of SHBG as shown in SEQ ID NO:2. Also preferred are fusion polypeptides comprising a sequence having a sequence similarity of at least 50%, preferred at least 60%, more preferred at least 70%, even more preferred at least 80% and most preferred 90% to SEQ ID NO:4 and further comprising the dimerization domain of SHBG.

Further preferred fusion polypeptides comprise anyone of the p75 fragments of human TNF receptor encoded by the sequences as set forth in SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, and SEQ ID NO:84 and the dimerization domain of SHBG.

A further preferred fusion polypeptide comprises the p55 fragment of human TNF receptor encoded by a nucleic acid sequence selected from the list consisting of SEQ ID NO:31, SEQ ID NO:32 and SEQ ID NO:33, and the dimerization domain of SHBG. In an especially preferred embodiment the fusion polypeptide consists of the p55 fragment of TNF receptor encoded by a nucleic acid sequence selected from the list consisting of SEQ ID NO:31, SEQ ID NO:32 and SEQ ID NO:33, and the dimerization domain of SHBG as shown in SEQ ID NO:2.

In another embodiment the fusion polypeptides comprise IL-1 and the dimerization domain of SHBG, IL-3 and the dimerization domain of SHBG, IL-6 and the dimerization domain of SHBG, or p55 TNF receptor (TNFR1a) or fragments thereof and the dimerization domain of SHBG.

The present invention is also directed to the use of the fusion polypeptides as a medicament. In a preferred embodiment the fusion polypeptide comprising the p75 fragment of TNF receptor as shown in SEQ ID NO:4 or a fusion polypeptide comprising a sequence having a sequence similarity of at least 50%, preferred at least 60%, more preferred at least 70%, even more preferred at least 80% and most preferred 90% to SEQ ID NO:4 is used as a medicament. These fusion polypeptides preferably further comprise the dimerization domain of SHBG.

In another preferred embodiment the fusion polypeptides comprising IL-1 and the dimerization domain of SHBG, IL-3 and the dimerization domain of SHBG, IL-6 and the dimerization domain of SHBG, or p55 TNF receptor (TNFR1a) or fragments thereof and the dimerization domain of SHBG are used as a medicament.

Another embodiment relates to fusion polypeptides of the invention for the treatment of diseases or disorders selected from the group consisting of autoimmune diseases, chronic inflammatory diseases, lymphoproliferative disorders, neurological and neuropsychiatric disorders.

In a preferred embodiment the fusion polypeptide comprising the p75 fragment of TNF receptor as shown in SEQ ID NO:4 or a fusion polypeptide comprising a sequence having a sequence similarity of at least 50%, preferred at least 60%, more preferred at least 70%, even more preferred at least 80% and most preferred 90% to SEQ ID NO:4 is used for the treatment of the above mentioned diseases. This fusion polypeptide preferably further comprises the dimerization domain of SHBG.

In another preferred embodiment the disease is selected from the group consisting of acute disseminated encephalomyelitis, addison's disease, alcohol withdrawal, Alzheimer's disease, amyotrophic lateral sclerosis (ALS), ankylosing spondylitis, anorexia nervosa, autism, autoimmune hepatitis, autoimmune oophoritis, B-Cell-Non-Hodgkin-lymphoma, Creutzfeldt-Jacob Disease (CJD), Variant CJD, Coeliac disease, Colitis Ulcerosa, Crohn's disease, depression, diabetes mellitus type 1, diabetic retinopathy, endometriosis, gestational pemphigoid, glaucoma, Goodpasture's syndrome, Graves disease, Guillain-Barré syndrome (GBS), Hashimoto's thyroiditis, Idiopathic Dementia, Idiopathic thrombocytopenic purpura (ITP), Kawasaki disease, Lewy Body Disease, Morbus Bechterew, Multiple Sclerosis, Muscular Dystrophies Myasthenia gravis, narcotic addiction, nicotine withdrawal, obsessive-compulsive disorder, Opsoclonus myoclonus syndrome (OMS), Optic neuritis, Parkinson's Disease, pemphigus, pernicious anemia, Pick's Disease, Polyarthritis, post-herpetic neuralgia, Psoriasis, primary biliary cirrhosis, rheumatoid arthritis (RA), Reiter's syndrome (reactive arthritis), Schizoaffective Illness, Schizophrenia, sepsis, Sjögrens syndrome, systemic lupus erythematodes (SLE), Takayasu's arteritis, Temporal arteritis, Unipolar and Bipolar Affective Disorders.

In a further preferred embodiment the fusion polypeptide comprising the p75 fragment of TNF receptor as shown in SEQ ID NO:4 or a fusion polypeptide comprising a sequence having a sequence similarity of at least 50%, preferred at least 60%, more preferred at least 70%, even more preferred at least 80% and most preferred 90% to SEQ ID NO:4 is used for the treatment of ankylosing spondylitis, Colitis Ulcerosa, Crohn's disease, systemic lupus erythematodes (SLE), Morbus Bechterew, Polyarthritis, rheumatoid arthritis (RA), Psoriasis, and/or Sjögrens syndrome. In an especially preferred embodiment this fusion polypeptide further comprises the dimerization domain of SHBG.

In a further especially preferred embodiment the fusion polypeptide comprising the p75 fragment of TNF receptor as shown in SEQ ID NO:4 or a fusion polypeptide comprising a sequence having a sequence similarity of at least 50%, preferred at least 60%, more preferred at least 70%, even more preferred at least 80% and most preferred 90% to SEQ ID NO:4 and the dimerization domain of SHBG is used for the treatment of endometriosis. The amino-terminal domain of human SHBG interacts with fibulin-1 and fibulin-2, two matrix-associated proteins located in the endometrium, and therefore targets SHBG to the endometrium and epididymis.

The present invention is also directed to pharmaceutical compositions comprising a fusion poypeptide of the present invention in a pharmacologically acceptable liquid, solid or semi-solid carrier, linked to a carrier or targeting molecule and/or incorporated into liposomes, microcapsules or a controlled release preparation or as nanoparticles.

The pharmaceutical compositions of the invention can be administered as any pharmaceutical formulation known in the art. Such pharmaceutical formulations comprise, for example, formulations which are suitable for oral, rectal, nasal or parenteral (including subcutaneous, intramuscular, and intravenous) administration. Preferably, the compositions are formulated for oral administration. More preferred the compositions are formulated for parenteral injection.

Normally, the pharmaceutical compositions of the present invention further comprise one or more pharmaceutical acceptable carrier that are physiologically compatible with the other substances comprised in the composition.

The compositions can be prepared according to standard methods which are well known in the art. See, e.g., Pharmaceutical Dosage Forms: Parenteral Medications Dekker, NY; Lieberman et al. (Hrsg.) (1990) Pharmaceutical Dosage Forms: Tablets Dekker, NY; und Lieberman et al. (Hrsg.) (1990) Pharmaceutical Dosage Forms: Disperse Systems Dekker, NY.

In particular, the pharmaceutical compositions of the present invention may contain a physiologically acceptable carrier together with the fusion polypeptides dissolved or dispersed therein as an active ingredient. As used herein, the term "pharmaceutically acceptable carrier" comprises, but is not limited to, water, saline, Ringer's Solutions, dextrose solution, and 5% human serum albumin. Liposome-based carriers and non-aqueous vehicles such as fixed oils may also be used. Further examples of suitable carriers for compositions comprising the fusion peptides are described in standard textbooks, for example, in "Remington's Pharmaceutical Sciences", Mack Pub. Co., New Jersey (1991). In addition to the carrier, the composition may also contain further compounds, such as wetting agents, emulsifying agents, pH buffering agents, stabilizers, dyes and the like, insofar as these compounds do not interfere with the biological activity of the fusion polypeptides of the invention. A stable liquid formulation can be prepared with or without lyophylisation process.

Preferably, the pharmaceutical composition is formulated for parenteral administration, for example, for intravenous, intradermal, subcutaneous, topical, transmucosal, or rectal administration. Solutions or suspensions used for parenteral, intradermal or subcutaneous application usually comprise a sterile diluent such as water, saline solution, fixed oils, polyethylene glycols, glycerine, propylene glycol or other synthetic solvents. The solutions or suspensions may further comprise antibacterial agents such as benzyl alcohol or methyl parabens, antioxidants such as ascorbic acid or sodium bisulfite, chelating agents such as EDTA, buffers such as acetates, citrates or phosphates, and agents for the adjustment of tonicity such as sodium chloride or dextrose. The pH can be adjusted with suitable acids or bases, such as hydrochloric acid or sodium hydroxide. The parenteral preparation can be enclosed in ampoules, disposable syringes or multiple dose vials made of glass or plastic.

Pharmaceutical compositions suitable for injection normally include sterile aqueous solutions or dispersions and sterile powders for the extemporaneous preparation of sterile injectable solutions or dispersion. The composition intended for injection must be sterile and should be fluid in order to allow a convenient handling in a syringe.

In one embodiment, the active compounds are prepared with carriers that will protect the fusion peptides against elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparing controlled release formulation are well-known in the art. Furthermore, sustained-release compositions can be prepared. Suitable examples of sustained-release preparations include semipermeable matrices of solid hydrophobic polymers, which matrices are in the form of shaped articles, e.g., films or microcapsules. Examples of sustained-release matrices include polyesters, hydrogels, polylactides, copolymers of L-glutamic acid and ethyl-L-glutamate, non-degradable ethylenevinyl acetate, degradable lactic acid-glycolic acid copolymers and the like.

The therapeutic composition of the present invention can include pharmaceutically acceptable salts of the components therein. As used herein, pharmaceutically acceptable salts include acid addition salts (formed with the free amino groups of the polypeptide) that are formed with inorganic acids such as, for example, hydrochloric or phosphoric acids, or such organic acids as acetic acid or tartaric acid and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like. Particularly preferred are the salts of TFA and HCl, when used in the preparation of cyclic peptides.

### Brief Description of the Figures:

- Figure 1:: The graph shows the result of a SEAP assay where only SHBG was used as a competitor. The competitor was used with increasing concentrations of 4nM, 20nM, 40nM and 200nM;
- Figure 2:: The graph shows the results of SEAP assays where the Pro_TNFαR1a_D41-L108_hinge_SHBG fusion polypeptide and Pro_TNFαR1a_D41-L108 without SHBG was used as a competitor. Each competitor was used with increasing concentrations of 4nM, 20nM, 40nM and 200nM;
- Figure 3:: The graph shows the results of SEAP assays where the Pro_TNFαR1a_D41-T127_hinge_SHBG fusion polypeptide and Pro_TNFαR1a_D41-T127 without SHBG was used as a competitor. Each competitor was used with increasing concentrations of 4nM, 20nM, 40nM and 200nM.

The present invention will now be described in greater detail with reference to the following examples. The following examples are for illustrative purposes and are not intended to limit the scope of the invention.

### EXAMPLES

### Example 1 Preparation of SHBG-TNF alpha receptor constructs

### a) Isolation of human SHBG cDNA clones and expression of SHBG peptides

A 1209 bp human SHBG cDNA was purchased from GeneCopoeia, Germantown, USA and used as a template to amplify a 615 bp SHBG cDNA fragment (SEQ ID NO:1) with the oligonucleotide primers SHBG 1205 F1 (5'-AACC**CATATG**GATACCAACC-3'; SEQ ID NO:23) and SHBG 1205 R1 (5'-GGTT**GGATCC**TCACGACCCAGAA-3'; SEQ ID NO:24). The NdeI recognition sequence (CATATG) and the BamHI recognition sequence (GGATCC) are shown in bold letters.

The reaction contained 0,2 mM of each dNTP, 75 µM of each primer, 10µg template, and 2,5 Units Pfu Turbo, Stratagene.

The PCR was carried out as a "Touch down PCR", i.e. the annealing temperature was lowered incrementally from 60°C to 50°C with 0,5 °C/Cycle, followed by 30 cycles at 50°C. PCR products were cloned blunt using the Zero Blunt TopoII PCR cloning kit, Invitrogen and TOP 10 competent cells, Invitrogen.

The 615 bp SHBG cDNA fragment was inserted in pET15b, Novagen, using BamHI and NdeI restriction enzymes, Fermentas GmbH, Germany, and ligase, Fermentas (method in Sambrook, E.F. Fritsch, T. Maniatis 2nd ed., New York : Cold Spring Harbour Laboratory Press, 1989).

For protein expression, BL21DE3 cells, Invitrogen, were transformed with the SHBG pET15b construct according Invitrogens user manual for One shot BL21(DE3) competent cells and grown at 37°C to an optical density at 600 nm wavelength (OD₆₀₀) of approximately 0,2. Protein expression was induced with 1mM IPTG (Isopropyl-β-D-thiogalactopyranosid) and grown for 4 hours to an OD₆₀₀ of approximately 2,5.

Bacteria were pelletised, resuspended to an OD₆₀₀ of 0,02, denatured and applied to an 4-12% Bis/Tris gel for expression control. Expression of a 22,5 kDa protein was visible.

### b) Isolation of human tumor necrosis factor α receptor (TNFR) 1a (p55) and TNFR 1b (p75) cDNAs

The sequences of human TNFαR1a (GenBank ACC-No. NM_00106) and TNFαR1b (GenBank ACC-No. NM_001066) were used to design the following synthetic oligonucleotide primers:

| | |
|---|---|
| TNFαR1a F1: | 5'-CCAAATGGGGGAGTGAGAGG-3' (SEQ ID NO: 25) |
| TNFαR1a R1: | 5'-TATGTACATCGAGGGGTTAGCA-3' (SEQ ID NO: 26) |
| | |
| TNFαR1b F1: | 5'-GAGGGCAGGGGGCAACC-3' (SEQ ID NO: 27) |
| TNFαR1b R1: | 5'-GGCGGGGCAGGTCACAGA-3' (SEQ ID NO: 28) |

The oligonucleotides TNFαR1a F1 and TNFαR1a R1 were used to amplify a 1544 bp TNFαR1a cDNA fragment (SEQ ID NO: 29) from a human spleen cDNA. A touch down PCR with incremental lowering of the annealing temperature by 0,5°C/cycle from 60°C to 50°C and subsequent 30 cycles at 50°C was performed. PCR conditions: 0,2 mM each dNTP, 75 µM each primer, 10µg template, 2,5 Units Pfu Turbo, Stratagene.

PCR products were cloned blunt using the Zero Blunt TopoII PCR cloning kit, Invitrogen and TOP 10 competent cells, Invitrogen.

The oligonucleotides TNFαR1b F1 and TNFαR1b R1 were used to amplify a 1718 bp TNFαR1b fragment (SEQ ID NO: 30) using a human spleen cDNA as template. A touch down PCR with incremental lowering of the annealing temperature by 0,5°C/cycle from 60°C to 50°C and subsequent 30 cycles at 50°C was performed. PCR conditions: 0,2 mM each dNTP, 75 µM each primer, 10µg template, 2,5 Units Pfu Turbo, Stratagene.

PCR products were cloned blunt using the Zero Blunt TopoII PCR cloning kit, Invitrogen and TOP 10 competent cells, Invitrogen.

### c) Preparation of TNFRαR1a- and TNFRαR1b-Ig_hinge-SHBG- constructs

I. In a first step, the nucleotide sequences TNFRαR1a D41-L108 (SEQ ID NO:31), TNFαR1a D41-T127 (SEQ ID NO:32), TNFαR1a D41-N161 (SEQ ID NO:33), TNFαR1b M1-C185 (SEQ ID NO:34), TNFαR1b P24-C185 (SEQ ID NO:84), TNFRαR1a M1-L108 (SEQ ID NO:93), TNFαR1a M1-T127 (SEQ ID NO:94), TNFαR1a M1-N161 (SEQ ID NO:95) and TNFαR1b S71-C126 (SEQ ID NO:3) were amplified from the TNFαR1a and TNFαR1b cDNAs using oligonucleotide primers designed for insertion in appropriate vectors for prokaryotic and eukaryotic expression, respectively. A touch down PCR with incremental lowering of the annealing temperature by 0,5°C/cycle from 60°C to 50°C and subsequent 30 cycles at 50°C was performed. PCR conditions: 0,2 mM each dNTP, 75 µM each primer, 10µg template, 2,5 Units Pfu Turbo, Stratagene.

The following oligonucleotide primers were used for the TNFαR1a constructs:

| | |
|---|---|
| pZB Pro L108: | |
| Pro_nde_F1: | 5'-AACC**CATATG**GATAGTGTGTGTCCCC-3' (SEQ ID NO:35) |
| Pro_L108_R1: | 5'-GGTT**GGATCC**TCAGAGGCTGCAATTGAAGC-3' (SEQ ID NO:36) |
| pZB Pro T127 Pro_nde_F1: | 5'-AACC**CATATG**GATAGTGTGTGTCCCC-3' (SEQ ID NO:35) |
| Pro_T127_R1: | 5'-GGTT**GGATCC**TCAGGTGCACACGGTGTTC-3' (SEQ ID NO:37) |
| pZB Pro N161 Pro_nde_F1: | 5'-AACC**CATAT**GGATAGTGTGTGTCCCC-3' (SEQ ID NO:35) |
| Pro_N161_R1 | 5'-GGTT**GGATCC**TCAATTCTCAATCTGGGGTAG-3' (SEQ ID NO:38) |
| pZB Euk L108R1 Euk_Hind F1: | 5'-AACC**AAGCTT**CCACCATGGGCCTCTCCACC-3' (SEQ ID NO:39) |
| Euk_L108_R1 | 5'-GGTT**GGATCC**TCAGAGGCTGCAATTGAAGC-3' (SEQ ID NO:40) |
| pZB Euk T127R1 Euk_Hind F1: | 5'-AACC**AAGCTT**CCACCATGGGCCTCTCCACC-3' (SEQ ID NO:39) |
| Euk_T127_R1 | 5'-GGTT**GGATCC**TCAGGTGCACACGGTGTTC-3' (SEQ ID NO:41) |
| pZB Euk N161R1 Euk_Hind F1: | 5'-AACC**AAGCTT**CCACCATGGGCCTCTCCACC-3' (SEQ ID NO:39) |
| Euk_N161_R1 | 5'-GGTT**GGATCC**TCAATTCTCAATCTGGGGTAG-3' (SEQ ID NO:42) |

The following oligonucleotide primers were used for the TNFαR1b constructs:
pZB Pro TNFαR1b_P24_C185:

| | |
|---|---|
| P75proF1 P75proR1 | 5'-AA**CATATG**CCCGCCCAGG-3' (SEQ ID NO:68) 5'-TT**GGATCC**TTAACAGATCTGGTGG-3' (SEQ ID NO:83) |
| pZB Euk TNFαR1b-M1-C185: | |
| P75F1 | 5'-AA**CTCGAG**GCCGCCACCATGGCGCCCGTCGC-3' (SEQ ID NO:43) |
| P75R1 | 5'-TT**GCGGCCGC**TTAACAGATCTGGTGG-3' (SEQ ID NO:44) |
| pZB Pro S71_C126: | |
| P75pro_S71_F: | 5'-AACC**CATATG**TCGGACACCGTGTGTG-3' (SEQ ID NO:45) |
| p75pro_C126_R: | 5'-GGTT**GGATCC**TCAgcagtaccagccgggcc -3' (SEQ ID NO:46) |
| pZB Euk S71_C126: | |
| 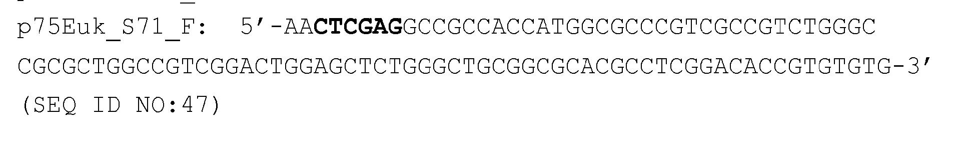 | |
| p75Euk_C126_R: | 5'-TT**GCGGCCGC**TCAgcagtaccagccgggcc-3' (SEQ ID NO:48) |

PCR products were cloned blunt using the Zero Blunt TopoII PCR cloning kit, Invitrogen and TOP 10 competent cells, Invitrogen.

For prokaryotic expression TNFαR1a fragments were inserted in pET24a, Novagen using the NdeI (CATATG) and BamHI (GGATCC) restriction sites in the oligonucletides.

Resulting constructs were:
pET24a_Pro_D41-L108
pET24a_Pro_D41-T127
pET24a_Pro_D41-N161

TNFαR1b_P24-C185 and TNFαR1b S71-C126 fragments were inserted in pET11a, Novagen using the NdeI (CATATG) and BamHI (GGATCC) restriction sites in the oligonucletides.

Resulting construct were:
pET11a_Pro_TNFαR1b_P24-C185
pET11a_TNFαR1b S71-C126

For eukaryotic expression TNFαR1a fragments were inserted in pcDNA3.1(+), Invitrogen using the BamHI (GGATCC) and HindIII (AAGCTT) restriction sites in the oligonucletides.

Resulting constructs were:
pcDNA3.1(+) Euk M1-L108
pcDNA3.1(+) Euk M1-T127
pcDNA3.1(+) Euk M1-N161

For preparation of TNFαR1b S71_C126 for eukaryotic expression, TNFαR1b S71_C126 construct was inserted in in pcDNA3.1(+), Invitrogen using the XhoI (CTCGAG) and NotI (GCGGCCGC) restriction sites in the oligonucleotides.

Resulting construct was:
pcDNA3.1(+)_TNFαR1b S71-C126

II. In a second step, an Ig hinge 87 nucleotide sequence (SEQ ID NO:21) was attached to the 3' end of the TNFαR1a and TNFαR1b cDNA fragments using chimeric oligonucleotide primers in an overlap PCR (Higuchi et al, 1988 Nucleic Acids Res 16 (15): 7351-67).

TNFR1a D41-L108, TNFR1a D41-T127, TNFR1a D41-N161 fragments were amplified using a "Touch down" PCR with incremental lowering of the annealing temperature by 0,5°C/cycle from 60°C to 50°C and subsequent 30 cycles at 50°C was performed. PCR conditions: 0,2 mM each dNTP, 75 µM each primer, 10µg template, 2,5 Units Pfu Turbo, Stratagene, with following oligonucleotides:
Pro_nde_F1 (SEQ ID NO:35) and Euk_Hind F1 (SEQ ID NO:39), respectively combined with L108_HingeR1 (5'-GGGTCTTGTCGCAGAGGCTGCAATTGAAGC-3' (SEQ ID NO:49)),
T127_HingeR1 (5'-GGGTCTTGTCGCAGGTGCACACGGTG-3' (SEQ ID NO:50)) and N161_HingeR1 (5'-GGGTCTTGTCGCAATTCTCAATCTGGGG-3' (SEQ ID NO:51)), respectively.

The resulting constructs are chimeras of TNFR1a L108 and Ig hinge partial sequence, TNFR1a T127 and Ig hinge partial sequence and TNFR1a N161 and Ig hinge partial sequence carrying NdeI or HindIII restriction sites at the 5'-end.

The resulting constructs were used together with a doublestranded Ig hinge sequence (SEQ ID NO:21) of 87 bp in an overlap PCR (5 cycles with annealing temperature of 50°C, followed by a touch down PCR with incremental lowering of the annealing temperature by 0,5°C/cycle from 60°C to 50°C and subsequent 30 cycles at 50°C), in which both fragments anneal and are combined by PCR with the both outer primers.

Outer primers were as follows:
Pro_nde_F1 (SEQ ID NO:35)
Euk_Hind F1 (SEQ ID NO:39)
Hinge_SHBGR1: 5'-CTTAGGGTTGGTATCCTTGGGCTTGGG-3' (SEQ ID NO:52)

III. In the next step, the SHBG fragment ((from a); see above) was fused to the 3' end of the TNFαR1a_hinge constructs (from the second step of c) using overlap PCR (PCR conditions as described above).

| | |
|---|---|
| PCR templates I: | Pro_L108-Hinge |
| | Pro_T127-Hinge |
| | Pro_N161-Hinge |
| | Euk_L108-Hinge |
| | Euk_T127-Hinge |
| | Euk_N161-Hinge |
| PCR template II | Hinge-SHBG |

Hinge-SHBG was amplified in a touch down PCR (Touch down PCR with incremental lowering of the annealing temperature by 0,5°C/cycle from 60°C to 50°C and subsequent 30 cycles at 50°C was performed. PCR conditions: 0,2 mM each dNTP, 75 µM each primer, 10µg template, 2,5 Units Pfu Turbo, Stratagene) with primers: Hinge_SHBG F1 and SHBG_BamHI R1:
Hinge_SHBG F1 5'-CCCAAGCCCAAGGATACCAACCCTAAG-3' (SEQ ID NO:53) SHBG_BamHI R1 5'-GGTTGGATCCTCACGACCCAGAAGACAACAC-3' (SEQ ID NO:54)
Each of the PCR templates I were combined with PCR template II resulting in an overlap PCR (PCR conditions as above).

Outer primers were Pro_nde_F1 (SEQ ID NO:35), Euk_Hind_F1 (SEQ ID NO:39) and SHBG_BamHI R1 (SEQ ID NO:54).

PCR products (= TNFαR1a_hinge_SHBG constructs) were cloned blunt using the Zero Blunt TopoII PCR cloning kit, Invitrogen, and TOP 10 competent cells, Invitrogen.

### TNFαR1a hinge SHBG pZB

Resulting products contain a TNFR1a fragment, a Ig hinge and a SHBG dimerization domain.

Prokaryotic expression constructs:
Pro_TNFαR1a D41-L108_hinge_SHBG (SEQ ID NO:55)
Pro_TNFαR1a D41-T127_hinge_SHBG (SEQ ID NO:57)
Pro_TNFαR1a D41-N161_hinge_SHBG (SEQ ID NO:59)

The fusion proteins encoded by these constructs were:
Pro_TNFαR1a D41-L108_hinge_SHBG prot (SEQ ID NO:56)
Pro_TNFαR1a D41-T127_hinge_SHBG prot (SEQ ID NO:58)
Pro_TNFαR1a D41-N161_hinge_SHBG prot (SEQ ID NO:60)

Eukaryotic expression constructs:
Euk_TNFαR1a M1-L108_hinge_SHBG (SEQ ID NO:61)
Euk_TNFαR1a M1-T127_hinge_SHBG (SEQ ID NO:63)
Euk_TNFαR1a M1-N161_hinge_SHBG (SEQ ID NO:65)

The fusion proteins encoded by these constructs were:
Euk_TNFαR1a M1-L108_hinge_SHBG prot (SEQ ID NO:62)
Euk_TNFαR1a M1-T127_hinge_SHBG prot (SEQ ID NO:64)
Euk_TNFαR1a M1-N161_hinge_SHBG prot (SEQ ID NO:66)

### TNFαR1b hinge SHBG expression constructs

For preparation of TNFαR1b_hinge_SHBG expression constructs, the the pET11a and pcDNA3.1(+) TNFαR1b constructs were combined with an Ig-hinge-SHBG-sequence in overlap PCRs as described above.

| | |
|---|---|
| Template I: | pET11a_Pro_TNFαR1b_P24-C185 |
| | pET11a_TNFαR1b S71-C126 |
| | pcDNA3.1(+)_TNFαR1b S71-C126 |
| Template II: | Ig hinge SHBG (SEQ ID NO:67) |

### TNFαR1b P24 C185 hinge SHBG:

Outer primer for pro_TNFαR1b P24_C185_hinge_SHBG

| | |
|---|---|
| P75proF1 | 5'-AA**CATATG**CCCGCCCAGG-3' (SEQ ID NO:68) |
| SHBG_BamHI R1 | 5'-GGTTGGATCCTCACGACCCAGAAGACAACAC-3' (SEQ ID NO:54) |

Overlap primer for TNFαR1b P24_C185_hinge_SHBG

| | |
|---|---|
| P75ENHSF1 | 5'-CCCACCAGATCTGTTGCGACAAGACCCACAC-3' (SEQ ID NO:85) |
| P75ENHSR1 | 5'-GTGTGGGTCTTGTCGCAACAGATCTGGTGGG-3' (SEQ ID NO:86) |

The resulting construct was:
Pro_TNFαR1b P24_C185_hinge_SHBG (SEQ ID NO:87)

The fusion protein encoded by this construct was:
Pro_TNFαR1b P24_C185_hinge_SHBG prot (SEQ ID NO:88)

TNFαR1b S71-C126 hinge SHBG:
Outer primer for pro_TNFαR1b S71-C126_hinge_SHBG

| | |
|---|---|
| P75proF1 | 5'-AA**CATATG**CCCGCCCAGG-3' (SEQ ID NO:68) |
| SHBG_BamHI R1 | (SEQ ID NO:54) |

Outer primer for euk_TNFαR1b S71-C126_hinge_SHBG
p75Euk_S71_F (SEQ ID NO:47)
SHBG_BamHI R1 (SEQ ID NO:54)

Overlap primer

| | |
|---|---|
| TNFR1b_C126_hinge_F | 5'-CCCGGCTGGTACTGCTGCGACAAGACCCACACC- 3' (SEQ ID NO:69 ) |
| TNFR1b_C126_hinge_R | 5'-GGTGTGGGTCTTGTCGCAGCAGTACCAGCCGGG- 3' (SEQ ID NO:70) |

The resulting constructs were:
Pro_TNFαR1b S71-C126_hinge_SHBG (SEQ ID NO:89)
euk_TNFαR1b S71-C126_hinge_SHBG (SEQ ID NO:91)

The fusion proteins encoded by these constructs were:
Pro_TNFαR1b S71-C126_hinge_SHBG prot (SEQ ID NO:90)
euk_TNFαR1b S71-C126_hinge_SHBG prot (SEQ ID NO:92)

TNFαR1b_hinge_SHBG constructs for prokyaryotic and eukaryotic expression: TNFαR1b S71-A127, TNFαR1b S71-L128, TNFαR1b S71-S129, TNFαR1b S71-K130, TNFαR1b S71-Q131, TNFαR1b S71-E132, TNFαR1b S71-G133, TNFαR1b S71-C134, TNFαR1b S71-R135, TNFαR1b S71-L136, TNFαR1b S71-C137, TNFαR1b S71-A138, TNFαR1b S71-P139, TNFαR1b S71-L140, TNFαR1b S71-R141, TNFαR1b S71-K142 were constructed in an analogous manner.

### d) Construction of an eukaryotic expression vector for expression of Euk_TNFαR1b_M1-C185 in mammalian cell lines.

pcDL-SRa296 vector (Takebe et al, 1988, MCB 8: 466-472) was used for construction of an expression vector containing DHFR as selection marker for transfection of CHO DHFR-cells.

Construction of pcDL-Sralpha296_II_IRES_DHFR:
An IRES (Internal ribosomal entry site) sequence and a mouse DHFR (Dihydrofolate reductase) sequence were amplified and combined in an overlap PCR using overlap oligonucleotides:

| | |
|---|---|
| IRDHFR2 | 5'-GTTCAATGGTCGAACCATTTATCATCGTGTTTTTC-3' (SEQ ID NO:71) |
| IRDHFR1 | 5'-GAAAAACACGATGATAAATGGTTCGACCATTGAAC-3' (SEQ ID NO:72) |

And outer oligonucleotides:

| | |
|---|---|
| IRNPT1 | 5'-GCTAGCCGCCCCTCTCCCTC-3' (SEQ ID NO:73) |
| IRDHFR3 | 5'-GGATCCTTAGTCTTTCTTCT-3' (SEQ ID NO:74) |

Resulting sequence was IRES mouse DHFR (SEQ ID NO:75) which was cloned NheI / BamHI in pcDL SR alpha 296 MCSII (SEQ ID NO:76).

This vector was used for subcloning of eukaryotic TNFR1b constructs, e.g., TNFR1b M1-C185 was cloned XhoI / NotI in pCDSRalpha 296 MCSII IRES mouse DHFR.

### Example 2 Expression of TNFαR1a_Ig_hinge_SHBG constructs

### a) Expression of TNFαR1a_Ig_hinge_SHBG in a prokaryotic expression system

The TNFαR1a_hinge_SHBG-inserts from clones: Pro_TNFαR1a D41-L108_hinge_SHBG_pZB, Pro_TNFαR1a D41-T127_hinge_SHBG_pZB and Pro_TNFαR1a D41-N161_hinge_SHBG_pZB were cloned in pET24, Novagen, (a brand of EMD Chemicals Inc., an Affiliate of Merck KGaA, Darmstadt, Germany) using BamHI and NdeI restriction enzymes, Fermentas and ligase, Fermentas (Method in Sambrook, E.F. Fritsch, T. Maniatis 2nd ed., New York : Cold Spring Harbour Laboratory Press, 1989).

For protein expression, BL21DE3 cells, Invitrogen, were transformed with the resulting constructs Pro_TNFαR1a D41-L108_hinge_SHBGpET24a, Pro_TNFαR1a D41-T127_hinge_SHBGpET24a and Pro_TNFαR1a D41-N161_hinge_SHBGpET24a according Invitrogens User Manual for One shot BL21(DE3) competent cells and grown in LB medium with 50 mg/l kanamycin sulfate at 37°C to an optical density at 600 nm wavelength (OD₆₀₀) of approximately 0,2. Protein expression was induced with 1mM IPTG (Isopropyl-β-D-thiogalactopyranosid) and cells were grown for another 4 hours to an OD₆₀₀ of approximately 3,0.

Cells were harvested by centrifugation (10 minutes, 4600 rpm, 4°C) and resuspended in Tris/EDTA-buffer (50mM Tris/HCl pH 8,5, 5mM EDTA) to an OD₆₀₀ of 0,02, denatured and applied to an 4-12% Bis/Tris gel for expression control. Gel was blotted to a Polyvinylidene Difluoride (PVDF) membrane. Protein expression was confirmed with Western Blot Analysis (Towbin et al. 1979, United State Patent 4840714) using anti-TNFαR antibody, Santa Cruz Biotechnology, INC according to manufacturers instructions.

Cell lysis was achieved by sonication (constant duty cycle, output control 3.5, eight times 20 seconds) and addition of 1% DOC (desoxycholate).

Protein was harvested by centrifugation (20 minutes, 15000 rpm, 4°C). Protein was denatured in denaturing buffer (100 mM Tris / NaOH, pH 13,0, 2M Urea) and renatured in renaturing buffer (Tris 50mM pH8,5; 0,5 mM EDTA; 2 M Urea; 10% Glycerine; 1 mM PMSF).

Purification of renatured protein using DEAE 5ml FF column and subsequent concentration using Centricon Plus-20 MWCo 10000, Millipore. Concentrated protein was applied to a size exclusion column Superdex 200 10/30 HR, AmershamPharmacia Biotech and eluted with Tris/HCl/Glycerol buffer pH 8,5 and Tris/HCl/NaCL/Glycerol buffer pH8,5.

### b) Expression of TNFαR1a_Ig_hinge_SHBG in a eukaryotic expression system

The TNFαR1a_hinge_SHBG-inserts from clones: Euk_TNFαR1a D41-L108_hinge_SHBGpZB, Euk_TNFαR1a D41-T127_hinge_SHBGpZB, Euk_TNFαR1a D41-N161_hinge_SHBGpZB were cloned in pcDNA3.1 (Invitrogen) using BamHI and HindIII restriction enzymes, Fermentas and ligase, Fermentas. The resulting constructs were:
Euk_TNFαR1a_D41-L108_hinge_SHBGpcDNA3
Euk_TNFαR1a_D41-T127_hinge_SHBGpcDNA3
Euk_TNFαR1a_D41-N161_hinge_SHBGpcDNA3

1,5*10⁶ CHO DG44 cells, dihydrofolate reductase deficient (dhfr⁻) derivates of Chinese Hamster Ovary (CHO) cells, were transfected with above pcDNA3 constructs (1,5 µg DNA) using Lipofectamine, Invitrogen and Optimem medium, Invitrogen according manufacturers instructions. After cultivation of the cells for 20 days protein expression was controlled by Western Blot Analysis of 1 ml cell suspension.

TNFαR1a_hinge_SHBG protein was enriched from the cell suspension by batch elution with Concanavalin A Sepharose 4B, Sigma-Aldrich (Vinogradova et al., Proc Natl Acad Sci U S A. 2000 Feb 15;97(4):1450-5).

Enriched protein was applied to an 4-12% Bis/Tris gel. Gel was blotted to a Polyvinylidene Difluoride (PVDF) membrane. Protein expression was confirmed with Western Blot Analysis (Towbin et al. 1979, Proc Natl Acad Sci U S A, Sep;76(9):4350-4; and US Patent No: 4840714) using anti-TNFαR antibody, Santa Cruz Biotechnology, INC according to manufacturers instructions.

For protein purification, TNFαR1a_hinge_SHBG protein was enriched from transiently or stably transfected cells by batch elution with Concanavalin A Sepharose 4B, Sigma-Aldrich (Vinogradova et al., 2000). Protein was applied to HiTrap DEAE 5ml FF column, eluted with 50 mM Tris-HCl pH 8,5, 5 mM EDTA, 1 M NaCl and subsequently concentrated using Centricon Plus-20 MWCo 10000, Millipore. Concentrated protein was applied to a Superdex 200 HR 10/30 column, GE Healthcare and eluted with 50 mM Tris-HCl pH 8,5, 5 mM EDTA.

### Example 3 Construction of HEK 293T cell line stably expressing SEAP NfkappaB

1,2*10⁵ HEK 293T cells were transfected with 0,8 µg DNA of PSEAP2basicNFkBNeo containing the SEAP (secreted alkaline phosphatase) cDNA under the control of the NF-κB promotor using Lipofectamine, Invitrogen and Optimem medium, Invitrogen according to manufacturers instructions. HEK cells were transfected and plated to a 24 well micro plate. After 24 hours medium was changed. After another 24 hours cells were stimulated with 1000 Units/well TNF alpha ligand (100,000 Units/µl), JenaBioscience and "homemade" TNF alpha ligand. Response to TNF alpha activation of TNF receptor was measured in the supernatant using the Great EscAPe SEAP (secreted form of human placental alkaline phosphatase) assay (Clontech). Responding pools were subjected to a G418 (neomycin) selection and cultivated in DMEM, PAA Laboratories containing 1000 µg G418, PAA Laboratories / ml medium.

### Cloning of TNF alpha ligand V77-L234

### PCR

The sequence of human TNF ligand superfamily member 2 (TNF SF2, Acc.No.NM_000594) was used to design synthetic oligonucleotides:

| | |
|---|---|
| hTNFaF1 | 5' ATCCCCTGACAAGCTGCCAGGCAGGTTCTC 3' (SEQ ID NO:77) |
| hTNFalphR1 | 5' AGGGGAGGCGTTTGGGAAGGTTGGATGTTC 3' (SEQ ID NO:78) |

A 823 bp TNF ligand superfamily member 2 fragment (SEQ ID NO:79) was amplified with oligonucleotides hTNFaF1 and hTNFalphR1 using a human spleen cDNA as template and cloned into the pH6ex6 vector.

A 474 bp TNF ligand superfamily member 2 fragment (SEQ ID NO:80) was amplified with oligonucleotides TNF V77 F1 and TNF V77 R1 using the TNF ligand pH6ex6 DNA and cloned into the pET 11a vector (Novagen).

| | |
|---|---|
| TNF V77 F1 | 5'-AAC**CATATG**GTCAGATCATCTTCTCGAAC-3'(SEQ ID NO:81) |
| TNF V77 R1 | 5'-GGTT**GGATCC**TCACAGGGCAATG-3' (SEQ ID NO:82) |

A touch down PCR with incremental lowering of the annealing temperature by 0,5°C/cycle from 60°C to 50°C and subsequent 30 cycles at 50°C was performed. PCR conditions: 0,2 mM each dNTP, 75 µM each primer, 10µg template, 2,5 Units Pfu Turbo, Stratagene.

PCR products were cloned blunt using the Zero Blunt TopoII PCR cloning kit, Invitrogen, and TOP 10 competent cells, Invitrogen: TNF_alpha_ligand_pZB

### Expression of TNF alpha ligand V77-L234 in a prokaryotic expression system:

The TNF alpha ligand V77-L234-inserts from TNF_alpha_ligand_pZB were cloned in pET11a, Novagen, using BamHI and NdeI restriction enzymes, Fermentas and ligase, Fermentas.

For protein expression, BL21DE3 cells, Invitrogen were transformed with the resulting construct TNF_alpha_ligand_pET11a according Invitrogens User Manual for One shot BL21(DE3) competent cells and grown in LB medium with 100 mg/l ampicillin at 37°C to an optical density at 600 nm wavelength (OD₆₀₀) of approximately 0,8. Protein expression was induced with 1mM IPTG (Isopropyl-β-D-thiogalactopyranosid) and cells were grown for another 3,5 hours to an OD₆₀₀ of approximately 3,0.

Cells were harvested by centrifugation (15 minutes, 4600 rpm, 4°C) and resuspended in Sodium phosphate buffer (50mM Sodium Phosphate pH7,4, 1mM EDTA, 1mM PMSF, 5% glycerol). Cell lysis was achieved by 4 sonication procedures (duty cycle 50%, output control 3.5, eight times 20 seconds).

The sample was clarified by centrifugation at 27,000 x g for 20 min at 4° C, then ammonium sulphate-precipitated at a saturation of 30%. The precipitate was dissolved in a small volume of 0.05 M sodium phosphate, pH 7.2, then re-precipitated with ammonium sulphate to a saturation of 50%. This second precipitate was dissolved in as small a volume as possible of 0.05 M sodium phosphate, pH 7.2, sterile filtered, then fractionated on a Superdex 200 prep grade HiLoad 16/60 column in 50 mM Tris-HCl, pH 8.0, 10% glycerol. Peak fractions were analysed by SDS-PAGE and Western blotting. TNF-alpha containing fractions were pooled.

Protein fractions were applied to HiTrap QFF column, eluted with 50 mM Tris-HCl pH 8,0, 10% glycerol, 0.5 M NaCl and subsequently concentrated using Centricon Plus-20 MWCo 10000, Millipore.

### SEAP assay

Stimulation of TNF receptors with TNF ligand leads to translocation of NF-κB to the nucleus, where it binds to a promotor sequence leading to transcription of SEAP (secreted alkaline phosphatase) cDNA. (Berger et al., Gene, 1988, 66:1-10). TNFR1a constructs were tested for their ability to inhibit the induction of NF-κB nuclear translocation and therefore inhibit secretion of AP using the Great EscAPe SEAP (secreted form of human placental alkaline phosphatase) assay (Clontech) according to manufacturers instructions. (Great EscAPe SEAP Reporter System 2. Clontechniques XI(4):6-7 (1996); New Fluorescent Great EscAPe SEAP Assay. Clontechniques XII(1):18-19 (1997)).

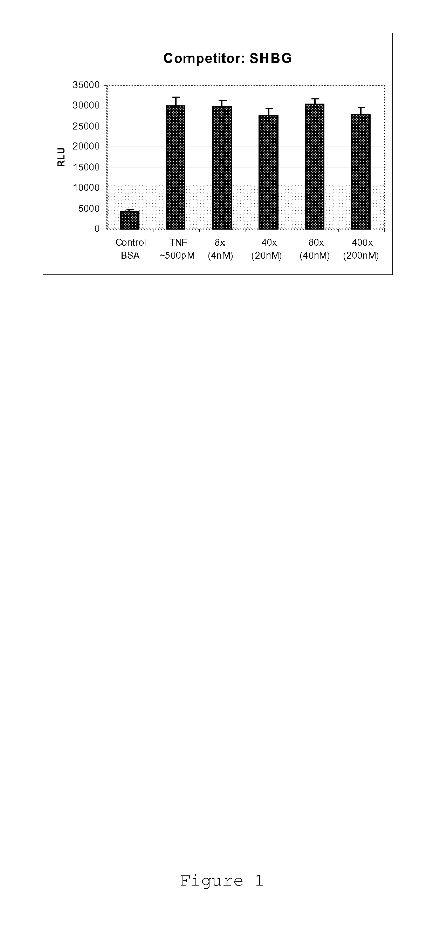

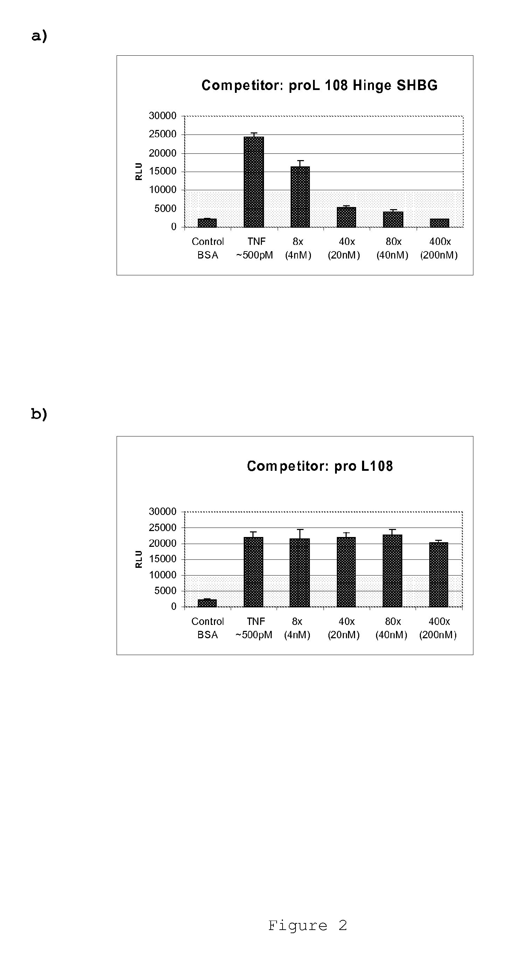

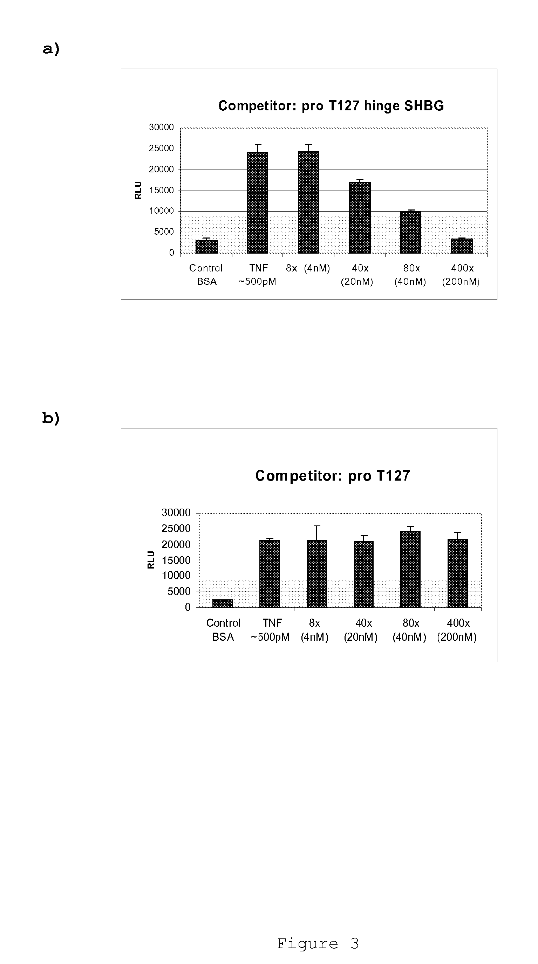

Fluorescence was measured using a Tecan SafireII Fluorescence reader.
a) In a first experiment the dimerization domain of SHBG was used as a competitor in an SEAP assay. The results are shown in Figure 1. Column 1 shows the fluorescence of the cells when stimulated with BSA as control. The stimulation with TNF ligand (column 2) led to a much higher fluorescence which is the result of the stimulation of the cells by TNFalpha. The addition of SHBG as a competitor in increasing concentrations (4nm, col. 3; 20nm, col.4; 40nM, col. 5; and 200nm, col. 6, respectively) had no influence on the stimulation of the cells by TNFalpha.
b) In a second experiment the Pro_TNFαR1a_D41-L108_hinge_SHBG fusion polypeptide was used as a competitor (Fig. 2a) and Pro_TNFαR1a_D41-L108 without SHBG was used as a control (Fig. 2b) in a SEAP assay. As can be clearly seen from Fig. 2a the Pro_TNFαR1a_D41-L108_hinge_SHBG fusion polypeptide led to a considerable decrease in the fluorescence of the cells (columns 3-6 compared with col. 2), which means that it inhibited the induction of NF-κB nuclear translocation and therefore the action of TNFalpha. Pro_TNFαR1a_D41-L108 without SHBG had no influence on the stimulation of the cells (Fig. 2b).
c) In a third experiment the Pro_TNFαR1a_D41-T127_hinge_SHBG fusion polypeptide was used as a competitor (Fig. 3a) and Pro_TNFαR1a_D41-T127 without SHBG was used as a control (Fig. 3b) in a SEAP assay. Similar to the results of the second experiment (item b), the fusion polypeptide including the SHBG dimerization domain led to a considerable decrease in the fluorescence while the polypeptide without SHBG had no effect.

## Claims

1. A nucleic acid molecule encoding a fusion polypeptide, which fusion polypeptide comprises the following fusion polypeptide components:
(a) a biologically active component; and
(b) a dimerization component,
which dimerization component (b) is capable of forming dimers with a dimerization component (b) comprised in another of said fusion polypeptides, wherein said dimerization component is derived from sex hormone-binding globulin (SHBG).

2. A nucleic acid molecule according to claim 1, wherein the biologically active component is a ligand-binding component, a substrate-binding component or a substrate component.

3. A nucleic acid molecule according to claim 1 or 2, wherein the encoded fusion polypeptide further comprises a linker peptide which is located between components (a) and (b) of the fusion polypeptide.

4. A nucleic acid molecule according to claim 3, wherein the linker peptide has a length of at least 10 amino acids up to 125 amino acids and is selected from the group consisting of Fc hinge, Ig hinge, endosomal escape domain, Glycin-linker, or fragments thereof.

5. A nucleic acid molecule according to claim 4, wherein the linker peptide is encoded by the nucleic acid sequence shown in SEQ ID NO:21.

6. A nucleic acid molecule according to any one of claims 1 to 5, wherein the nucleic acid encoding the dimerization component derived from sex hormone-binding globulin (SHBG)
a) comprises the nucleic acid sequence as shown in SEQ ID NO:1; and/or
b) comprises a fragment of SEQ ID NO:1 of at least 50 nucleotides; and/or
c) has a nucleotide acid sequence similarity of at least 80%, preferred 90% and most preferred 95% to SEQ ID NO:1.

7. A nucleic acid molecule according to any one of claims 1 to 6, wherein the dimerization component derived from sex hormone-binding globulin (SHBG) is capable of forming dimers with a dissociation constant K_{D} of at least 10⁻⁹, preferably at least 10⁻¹⁰, more preferably at least 10⁻¹¹ and most preferred at least 10⁻¹².

8. A nucleic acid molecule according to any one of claims 1 to 7, wherein the dimerization component derived from sex hormone-binding globulin (SHBG) is a chimeric construct consisting of at least two parts of the dimerization domain which are naturally not adjacent to each other.

9. A nucleic acid molecule according to any one of claims 1 to 7, wherein the dimerization component derived from sex hormone-binding globulin (SHBG) is a chimeric construct consisting of at least two parts, one of which is derived from the dimerization domain of SHBG, whereas the at least one further part is derived from another protein.

10. A nucleic acid molecule according to claim 9, wherein the at least one further part is derived from cortisol-binding globulin (CBG).

11. A nucleic acid molecule according to any one of claims 1 to 10, wherein the biologically active component is selected or derived from the group consisting of IL-1, IL-1R, IL-2, IL-2R, IL-3, IL-3R, IL-4, IL-4R, IL-5, IL-5R, IL-6, IL-6R, IL-7, IL-7R, IL-8, IL-9, IL-9R, IL-10, IL-11, IL-12, IL-13, IL-13R, IL-14, IL-15, IL-15R, IL-16, IL-17, TNF, TGF, TGF-α, IFN, GM-CSF, GM-CSFR, G-CSF, G-CSFR, EPO, EPOR, TPO, M-CSF, GHR, TNFR, TGFR, IFNR, interferon-α R, interferon-β R, interferon-γ R, cMp1, gp130, Fas (Apo 1), CCR1, CXCR1-4, TrkA, TrkB, TrkC, Htk, REK7, Rse/Tyro-3, hepatocyte growth factor R, platelet-derived growth factor R, Flt-1, CD2, CD4, CD5, CD6, CD22, CD27, CD28, CD30, CD31, CD40, CD44, CD100, CD137, CD150, LAG-3, B7, B61, β-neurexin, CTLA-4, ICOS, ICAM-1, complement R-2 (CD21) IgER, lysosomal mebrane gp1, α2 microglobulin receptor related proteins and sodium-releasing peptide R, LIF, LT, FGF, VEGF, EGF, SCF, oncostatin M, Amphiregulin, Mullerian-inhibiting substance, BCGF, MIF, Endostatin and Angiostatin, GLP, GLP2, PACAP, VIP, CD4 cluster of differentiation, Secretin, glicentin, Oxyntomodulin, ANP, BNP, Interferone, BDNF, NGF, GDNF, Somatostatin, enzymes from the group of hydrolases (including phosphatases, nucleases phosphodiesterases), oxidoreductases, transferases (including phosphotransferases), lyases, isomerases and ligases and substrates of these enzymes including nucleotides, proteins, or biologically active fragments, variants, mutants, analogs or derivatives thereof.

12. An expression vector comprising a nucleic acid molecule of any one of claims 1 to 11.

13. A host cell comprising the expression vector of claim 12.

14. The host cell of claim 13, wherein the host cell is a bacterial cell, yeast cell, insect cell, or mammalian cell.

15. The host cell of claim 14, wherein the host cell is E. coli, a CHO cell, a 293 cell, or a K562 cell.

16. A fusion polypeptide encoded by the nucleic acid molecule according to any one of claims 1 to 11.

17. A fusion polypeptide in dimeric form comprising two fusion polypeptides according to claim 16.

18. A method of producing the fusion polypeptide of claim 16 or 17 which comprises growing the host cell of claims 13 to 15 under conditions permitting production of the fusion polypeptides and recovering the fusion polypeptides so produced.

19. A monomeric or dimeric fusion polypeptide obtained by the method of claim 18.

20. A pharmaceutical composition comprising a fusion polypeptide of claims 16, 17 or 19 in a pharmacologically acceptable liquid, solid or semi-solid carrier, linked to a carrier or targeting molecule and/or incorporated into liposomes, microcapsules or a controlled release preparation or as nanoparticles.

21. A fusion polypeptide according to claims 16, 17 or 19 for use as a medicament.

22. A fusion polypeptide according to claims 16, 17 or 19 for the treatment of diseases or disorders selected from the group consisting of autoimmune diseases, chronic inflammatory diseases, lymphoproliferative disorders, neurological and neuropsychiatric disorders, and cancer.

23. A fusion polypeptide according to claim 22, wherein the disease is selected from the group consisting of acute disseminated encephalomyelitis, addison's disease, alcohol withdrawal, Alzheimer's disease, amyotrophic lateral sclerosis (ALS), ankylosing spondylitis, anorexia nervosa, autism, autoimmune hepatitis, autoimmune oophoritis, B-Cell-Non-Hodgkin-lymphoma, Creutzfeldt-Jacob Disease (CJD), Variant CJD, Coeliac disease, Colitis Ulcerosa, Crohn's disease, depression, diabetes mellitus type 1, diabetic retinopathy, endometriosis, gestational pemphigoid, glaucoma, Goodpasture's syndrome, Graves disease, Guillain-Barré syndrome (GBS), Hashimoto's thyroiditis, Idiopathic Dementia, Idiopathic thrombocytopenic purpura (ITP), Kawasaki disease, Lewy Body Disease, Morbus Bechterew, Multiple Sclerosis, Muscular Dystrophies Myasthenia gravis, narcotic addiction, nicotine withdrawal, obsessive-compulsive disorder, Opsoclonus myoclonus syndrome (OMS), Optic neuritis, Parkinson's Disease, pemphigus, pernicious anemia, Pick's Disease, Polyarthritis, post-herpetic neuralgia, Psoriasis, primary biliary cirrhosis, rheumatoid arthritis (RA), Reiter's syndrome (reactive arthritis), Schizoaffective Illness, Schizophrenia, sepsis, Sjögrens syndrome, systemic lupus erythematodes (SLE), Takayasu's arteritis, Temporal arteritis, Unipolar and Bipolar Affective Disorders.
